Europäisches Patentamt

European Patent Office

Office européen des brevets

(11) Publication number: **0 265 778**
**A1**

# EUROPEAN PATENT APPLICATION

(21) Application number: **87115043.9**

(22) Date of filing: **14.10.87**

(51) Int. Cl.⁴: **C12N 15/00** , C12N 1/00 , C12P 21/02 , A61K 37/02 , C07K 13/00

The microorganism has been deposited with the American Type Culture Collection under number ATCC 67111, 67236.

(30) Priority: **15.10.86 US 919153**

(43) Date of publication of application:
**04.05.88 Bulletin 88/18**

(84) Designated Contracting States:
**AT BE CH DE ES FR GB IT LI LU NL SE**

(71) Applicant: **RORER INTERNATIONAL (OVERSEAS) INC.**
**P.O. Box 145**
**Lewes, DE 19958(US)**

(72) Inventor: **Sarver, Nava**
**8122 Tuckerman Lane**
**Potomac Maryland(US)**
Inventor: **Drohan, William**
**5232 Perth Court**
**Springfield Virginia(US)**

(74) Representative: **Patentanwälte Grünecker, Kinkeldey, Stockmair & Partner**
**Maximilianstrasse 58**
**D-8000 München 22(DE)**

(54) **Factor VIII-C analogs.**

(57) Analogs of Factor VIII-C and their recombinant DNA directed synthesis are disclosed. These analogs have use in the treatment of coagulation disorders.

EP 0 265 778 A1

ASSEMBLY OF FULL-LENGTH FVIII cDNA

# FIG.3

B = BAMHI
H = HIND3
N = NDEI
P = PVU2
SA = SACI
SI = SIUI

1a.

## FACTOR VIII-C ANALOGS

This invention relates to the field of structural gene cloning and the use of such genes in the recombinant DNA directed synthesis of desired protein products. More particularly, it relates to new analogs of Factor VIII procoagulant activity protein (Factor VIII-C), its recombinant DNA directed synthesis and use in the treatment of coagulation disorders, such as hemophilia.

In general, recombinant DNA techniques are known. See Methods in Enzymology, (Academic Press) Volumes 65 and 68 (1979); 100 and 101 (1983) and the references cited therein, all of which are incorporated herein by reference. An extensive technical discussion embodying most commonly used recombinant DNA methodologies can be found in Maniatis, et al., Molecular Cloning, Cold Spring Harbor Laboratory (1982). Genes coding for various polypeptides may be cloned by incorporating a DNA fragment coding for the polypeptide in a recombinant DNA vehicle, e.g., bacterial or viral vectors, and transforming a suitable host, typically an Escherichia coli (E. coli) cell line, and isolating clones incorporating the recombinant vectors. Such clones may be grown and used to produce the desired polypeptides on a large scale.

Mixtures of mRNA from eukaryotic cells employing a series of three enzymatic reactions to synthesize double-stranded DNA copies of entire genes which are complementary to this mRNA mixture have been isolated. In the first reaction, mRNA is transcribed to form a single-strand complementary DNA (ss-cDNA) by an RNA-directed DNA polymerase, also called reverse transcriptase. Reverse transcriptase synthesizes DNA in the 5'-3'direction, utilizes deoxyribonucleoside 5'-triphosphates as precursors, and requires both a template and a primer stand, the latter of which must have a free 3'-hydroxyl terminus. Reverse transcriptase products, whether partial or complete copies of the mRNA template, often possess short, partially double-stranded hairpins ("loops") at their 3' termini. In the second reaction, these "hairpin loops" can be exploited as primers for DNA polymerases. Preformed DNA is required both as a template and as a primer in the action of DNA polymerase. The DNA polymerase requires the presence of a DNA strand having a free 3'-hydroxyl group, to which new nucleotide residues are added to extend the chain in the 5'-3' direction. The products of such sequential reverse transcriptase and DNA polymerase reactions still possess a loop at one end. The apex of the loop or "fold-point" of the double-stranded DNA, which has thus been created, is substantially a single-strand segment. In the third reaction, this single-strand segment is cleaved with the single-strand specific nuclease S1 to generate a "blunt-end" duplex DNA segment. This general method is applicable to any mRNA mixture, and is described by Buell, et al., J. Biol. Chem. 253:2483 (1978),

The resulting double-stranded cDNA (ds-cDNA) mixture is inserted into cloning vehicles by any one of many known techniques, depending at least in part on the particular vehicle being used. Various insertion methods are discussed in considerable detail in Methods In Enzymology, 68:16-18, and the references cited therein.

Once the DNA segment is inserted, the cloning vehicle is used to transform a suitable host. These cloning vehicles usually impart an antibiotic resistance trait on the host. Such hosts are generally prokaryotic or eukaryotic cells. At this point, only a few of the transformed or transfected hosts contain the desired cDNA. The sum of all transformed or transfected hosts constitutes a gene "library". The overall ds-cDNA library created by this method provides a representative sample of the coding information present in the mRNA mixture used as the starting material.

If an appropriate oligonucleotide sequence is available, it can be used to identify clones of interest as follows. Individual transformed or transfected cells are grown as colonies on nitrocellulose filter paper. These colonies are lysed, the DNA so-released is covalently attached to the filter paper by heating, and the sheet is incubated with a labeled oligonucleotide probe. The probe sequence is complementary to the structural gene of interest. The probe hybridizes with the ds-cDNA for which it is complementary and is identified by autoradiography. The clones are characterized to identify a clone containing all the structural information for the desired protein. The nucleic acid sequence coding for the protein of interest is isolated and reinserted into an expression vector. The expression vector brings the clones gene under the regulatory control of a specific prokaryotic or eukaryotic control element which allows the efficient expression (transcription and translation) of the cloned full-length ds-cDNA. Thus, this general technique is only applicable to those proteins for which at least a portion of their amino acid or DNA sequence is known and for which an oligonucleotide probe is available. See, generally, Maniatis, et al., supra.

More recently, methods have been developed to identify specific clones by probing bacterial colonies with antibodies specific for the encoded protein of interest. This method can only be used with "expression vector" cloning vehicles since elaboration of the product protein is required. The structural gene is inserted into the vector adjacent to the regulatory gene lysed either by the vector or by chemical methods, and the protein detected by the specific antibody and a labeling system, e.g., enzyme immunoassay. An example of this is the lambda gt₁₁ system described by Young and Davis, Proc. Nat'l. Acad. Sci. USA, 80:1194-1198 (1983) and Young and Davis, Science, 22:778 (1983).

Normal human blood plasma contains a complex of two proteins which is referred to as the Factor VIII complex. One component of the Factor VIII complex has antihemophilic factor procoagulant activity and is designated Factor VIII-C. A deficiency in Factor VIII-C is characteristic of hemophilia, a disease transmitted by X-chromosomal inheritance.

There is little information in the literature about Factor VIII-C analogs. Analogs to Factor VIII-C can be characterized by differences in the nucleic acid or amino acid sequence of Factor VIII-C as published in Gitschier, et al., [Nature, 312:326-330 (1984)] and by Wood, et al., [Nature, 312:330-337 (1984)]. Such analogs may arise from a mutation in the genetic code, from alternative mRNA splicing, or from post-translational modifications. No such Factor VIII-C analogs have been heretofore isolated or genetically engineered.

Factor VIII-C has therapeutic value because of its ability to correct the clotting deficiency in hemophilic patients. Unfortunately, the available methods of obtaining Factor VIII-C are limited to fractionation of human plasma, as described above. Producing Factor VIII-C by plasma fractionation provides only limited quantities which vary from preparation to preparation, are expensive, and subject the recipient hemophiliac to the risk of acquiring blood transmitted diseases such as hepatitis and acquired immune deficiency syndrome (AIDS).

Factor VIII-C analogs could provide an alternative agent to be used in the treatment of hemophilia. Analogs may prove more efficient than native Factor VIII-C in restoring a normal clotting cascade. Secondly, production of Factor VIII-C analogs via recombinant DNA technology would reduce the disease-transmission risks associated with plasma-fractionated products. Whereas the administration of a genetically engineered analog, whose secondary or tertiary structure is sufficiently different from native Factor VIII-C, may result in an immune response, native analogs are not expected to trigger this response. Genetically engineered analogs to Factor VIII-C, therefore, could provide a dependable and readily available therapeutic agent to be used in the treatment of hemophilia.

In recognizing and overcoming the above-described problems, the present invention has made it possible to provide readily available large quantities of Factor VIII-C analogs. This has been achieved with nucleic acids and antibodies which react specifically with the Factor VIII-C analog coding sequence of the Factor VIII-C analog protein molecule or the Factor VIII-C protein molecule (or fragments thereof); with the application of recombinant DNA technology to preparing cloning vehicles encoding for the Factor VIII-C analogs and screening/isolating procedures for recovering human Factor VIII-C analogs essentially free of other proteins of human origin.

Accordingly, the present invention provides human Factor VIII-C analogs or those fragments essentially free of other proteins of human origin. The Factor VIII-C analogs are produced by recombinant DNA techniques in host cells or other self-replicating systems and are provided in essentially pure form. Also provided are methods and compositions for preparing the above-described Factor VIII-C analogs as well as therapeutic compositions and uses for the Factor VIII-C analogs or subunit, in the treatment of coagulation disorders in humans and animals.

The invention further provides replicable expression vectors incorporated with a DNA sequence encoding Factor VIII-C analogs and host cell or cell-free self-replicating systems transformed or transfected thereby. The host system is usually of prokaryotic origin, e.g. E. coli or B. subtilis, or eukaryotic cells.

The Factor VIII-C analogs are produced by a process which comprises (a) preparing a replicable expression vector capable of expressing the DNA sequence encoding a Factor VIII-C analog in a suitable host cell or cell-free replicating system; (b) transforming said host system to obtain a recombinant host system; (c) maintaining said recombinant host system under conditions permitting expression of said Factor VIII-C analog encoding the DNA sequence to produce Factor VIII-C analog. The Factor VIII-C analog encoding replicable expression vector is made from a double-stranded complementary DNA (ds-cDNA) preparation representative of a messenger RNA pool containing messenger RNA for Factor VIII-C and incorporating DNA from the ds-cDNA pool into replicable expression vectors. The preferred mode of recovering Factor VIII-C analogs comprises reacting the proteins expressed by the recombinant host system with a reagent composition comprising at least one binding protein specific for Factor VIII-C analog.

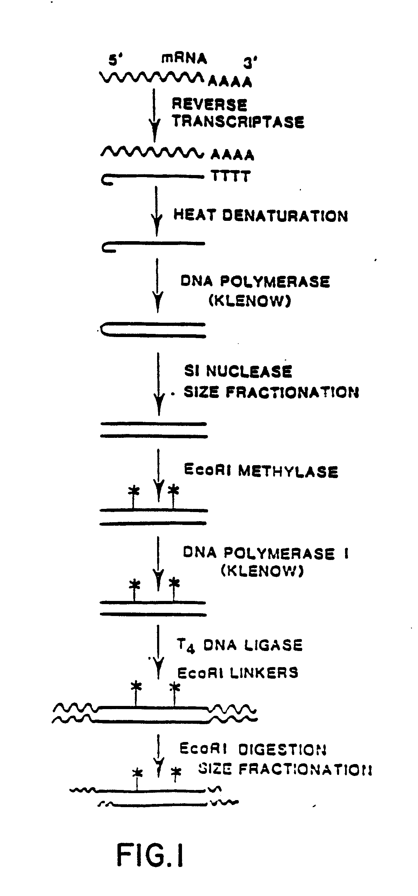

In the accompanying drawings, Figure 1 illustrates the preparation of cDNA from mRNA.

Figure 2 illustrates the preparation of a cDNA library and subsequent screening for FVIIIC specific clones.

Figure 3 diagrams the assembly of full length Factor VIII-C.

Figure 4A illustrates the design of a Factor VIII protein.

Figure 4B illustrates the complete Factor VIII cDNA and the resulting variant.

Figures 5-9 illustrates the DNA sequence of assembled full-length Factor VIII-C.

Figures 10-14 illustrate the DNA sequence of naturally occurring Factor VIII-C analog with Exon 4 deletion.

Figures 15-18 illustrate the DNA sequence of genetically engineered Factor VIII-C analog with a major portion of Exon 14 deleted.

Figures 19-22 illustrate the DNA sequence of genetically engineered Factor VIII-C analog with Exon 4 and 14 deletions.

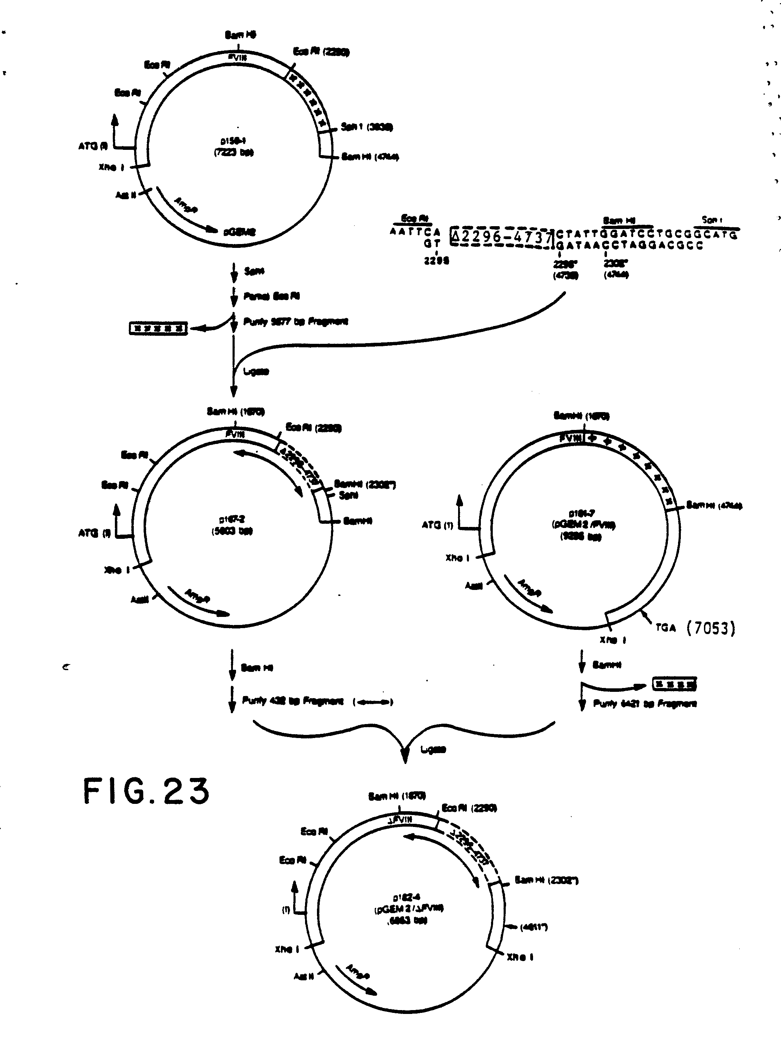

Figure 23 illustrates plasmids used in the invention.

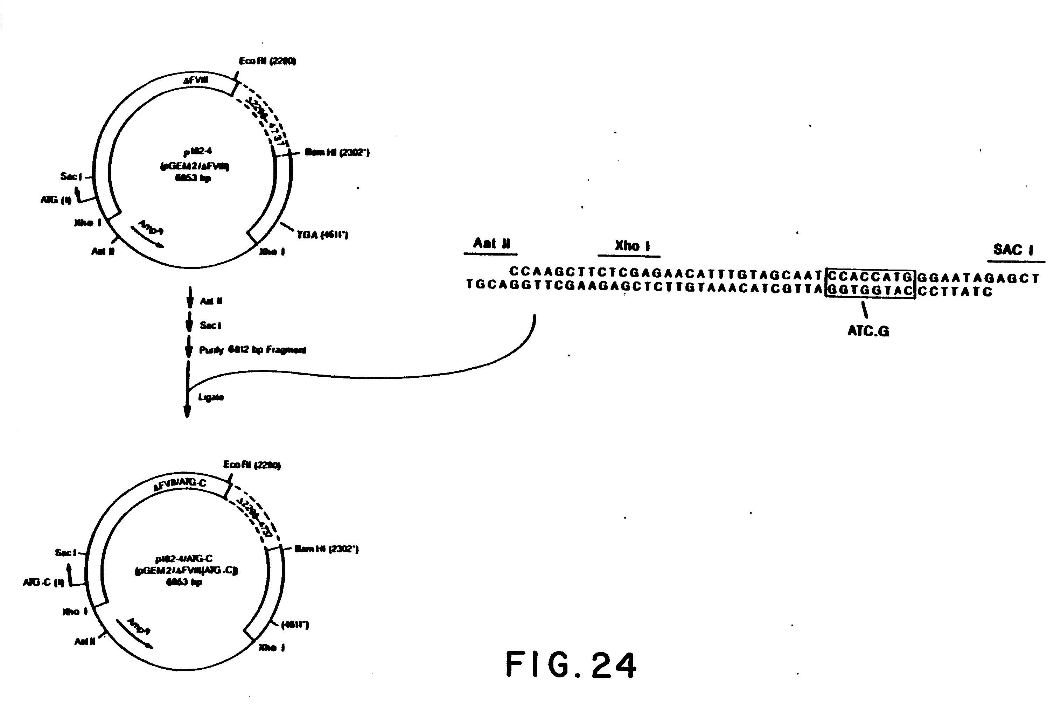

Figure 24 illustrates plasmid p182-4-ATG-C.

Figure 25 illustrates plasmids p-261-26 and p188-3.

As used herein, "Factor VIII-C analog" denotes human Factor VIII-C analogs, or subunits thereof, produced in in vitro cell culture systems, in bioactive forms having the capacity to initiate blood coagulation as does Factor VIII-C native to human plasma.

Analog of Factor VIII-C analogs may exist in nature. These variations may be characterized by difference(s) in the nucleotide sequence of the structural gene coding for proteins of identical biological function. These variations may also rise from alternate mRNA splicing patterns. It is possible to engineer analogs having single or multiple amino acid substitutions, deletions, additions, or replacements. All such variations, modifications, and analogs (naturally derived, synthetic or genetically engineered) resulting in derivatives of Factor VIII-C which retain the biologically active properties of native Factor VIII-C are included within the scope of this invention. The analog may be a recombinant-derived protein identical to analogs identified in cDNA libraries or newly engineered analogs with no counterparts in vivo.

"Expression vectors" refer to vectors which are capable of transcribing and translating DNA sequences contained therein, where such sequences are linked to other regulatory sequences capable of effecting their expression. These expression vectors must be replicable in the host organisms or systems either as episomes, bacteriophage, or as an integral part of the chromosomal DNA. One expression vector which is particularly suitable for producing human Factor VIII-C analogs is the bacteriophage, viruses which normally inhabit and replicate in bacteria. Particularly desirable phages for this purpose are the lambda gt10 and gt11 phage, described by Young and Davis, supra . Lambda gt11 is a general recombinant DNA expression vector capable of producing polypeptides specified by the inserted DNA.

To minimize degradation, upon induction with a synthetic analog of lactose (IPTG), foreign proteins or portions thereof are synthesized fused to the prokaryotic protein B-galactosidase. The use of host cells defective in protein degradation pathways may also increase the lifetime of novel proteins produced from the induced lambda gt11 clones. Proper expression of foreign DNA in lambda gt11 clones depends upon the proper orientation and reading frame of the inserted DNA with respect to the B-galactosidase promoter and ribosome binding site.

Another form of expression vector useful in recombinant DNA techniques is the plasmid - a circular, unintegrated (extra-chromosomal), double-stranded DNA loop. Any other form of expression vector which serves an equivalent function is suitable for use in the process of this invention.

Recombinant vectors and methodology disclosed herein are suitable for use in host cells covering a wide range of prokaryotic and eukaryotic organisms. Prokaryotics are preferred for the cloning of DNA sequences and in the construction of vectors. For example, E. coli K12 strain HB101 (ATCC No. 33694) is particularly useful. Of course, other microbial strains may be used, vectors containing replication and control sequence which are derived from species compatible with the host cell or system are used in connection with these hosts. The vector ordinarily carries an origin of replication, as well as characteristics capable of providing phenotypic selection in transformed cells. For example, E . coli can be transformed using the vector pBR322, which contains genes for ampicillin and tetracycline resistance [Bolivar, et al., Gene, 2:95 (1977)].

These antibiotic resistance genes provide a means of identifying transformed cells. The expression vector may also contain control elements which can be used by the vector for expression of its own proteins. Common prokaryotic control elements used for expression of foreign DNA sequences in E. coli include the promoters and regulatory sequences derived from the B-galactosidase and tryptophan (trp) operons of E. coli, as well as the pR and pL promoters of bacteriophage lambda. Combinations of these elements have also been used (e.g., TAC, which is a fusion of the trp promoter with the lactose operator). Other promoters have also been discovered and utilized, and details concerning their nucleotide sequences have been published enabling a skilled worker to combine and exploit them functionally.

In addition to prokaryotes, eukaryotic microbes, such as yeast cultures, may also be used. Saccharomyces cerevisiae, or common baker's yeast, is the most commonly used among eukaryotic microorganisms, although a number of other strains are commonly available. Suitable promoting sequences in yeast vectors include the promoters of 3-phosphoglycerate kinase or other glycolytic enzymes. Suitable expression vectors may contain termination signals which provide for the polyadenylation and termination of the cloned gene's mRNA. Any vector containing a yeast-compatible promoter, origin of replication, and appropriate termination sequence is suitable for expression of Factor VIII-C analogs.

In addition to microorganisms, cultures of cells derived from multicellular organisms may also be used as hosts. In principle, any such cell culture is workable, whether from a vertebrate or invertebrate source. However, interest has been greatest in vertebrate cells and propation of vertebrate cells in culture (tissue culture) has become a routine procedure in recent years. Examples of such useful hosts are the VERO, HeLa, mouse C127, Chinese hamster ovary (CHO), W138, BHK, COS-7, and MDCK cell lines. Expression vectors for such cells ordinarily include an origin of replication, a promoter located in front of the gene to be expressed, along with any necessary ribosome binding sites, RNA splice sites, polyadenylation site, and transcriptional terminatory sequence.

For use in mammalian cells, the control functions on the expression vectors are often provided by viral material. For example, commonly used promoters are derived from polyoma, Adenovirus 2, and most frequently, Simian Virus 40 (SV40). Further, it is also possible, and often desirable, to utilize promoter or control sequences naturally associated with the desired gene sequence, provided such control sequences are compatible with the host system. To increase the rate of transcription, eukaryotic enhancer sequences can also be added to the construction. These sequences can be obtained from a variety of animal cells, animal viruses or oncogenic retroviruses such as the mouse sarcoma virus.

An origin of replication may be provided either by construction of the vector to include an exogenous origin, such as that provided by SV40 or other viral sources, or may be provided by the host cell chromosomal replication mechanism. If the vector is integrated into the host cell chromosome, the latter is sufficient.

Specific to the example contained herein is the bovine papillomavirus (BPV). Use of BPV DNA as a viral vector is well documented and was originally based on the observation that the viral genome persists as an extrachromosomal plasmid in transformed cells (Law. M.-F., D.R. Lowy, I. Dvoretzky, and P.M. Howley, Proc. Natl. Acad. Sci. USA, 78:2727-2731, 1981). In this form, the cloned gene is maintained in a uniform sequence environment of the BpV minichromosome, eliminating potential problems associated with the integration of the cloned DNA into inactive regions of the host chromosome. This property was fully exploited in the establishment of BPV shuttle vectors capable of replicating in prokaryotic and eukaryotic cells [Sarver, N., J.C. Byrne, and P.M. Howley, Proc. Natl. Acad . Sci. USA, 79(13):4030-4034, 1982; and Kushner, P.J., B.B. Levinson, and H.M. Goodman, J. Mol. Appl. Genet., 1(6):527-528, 1982]. Such vectors are efficient in directing the regulated expression from inducible promoters and in expressing gene products destined for intra-and extracellular location. Vectors which may be employed as starting materials useful for the construction of the vectors of the subject invention are described in U.S. Patent 4,419, 446, the contents of which are incorporated herein by reference.

Host cells can prepare human Factor VIII-C analogs which can be of a variety of chemical compositions. The protein is produced having methionine as its first amino acid (present by virtue of the ATG start signal codon naturally existing at the origin of the structural gene or inserted before a segment of the structural gene). The protein may also be intra-or extracellularly cleaved, giving rise to the amino acid which is found naturally at the amino terminus of the protein. The protein may be produced together with either its signal polypeptide or a conjugated protein other than the conventional signal polypeptide; the signal polypeptide of the conjugate being specifically cleavable in an intra-or extracellular environment. Finally, Factor VIII-C analogs may be produced by direct expression in mature form without the necessity of cleaving away any extraneous polypeptide.

Recombinant host cells are cells which have been transformed with vectors constructed using recombinant DNA techniques. As defined herein, Factor VIII-C analogs are produced as a consequence of this transformation. Factor VIII-C analogs or its subunits produced by such cells are referred to as "recombinant Factor VIII-C analogs".

The procedures below are but some of a wide variety of well-established procedures to produce specific reagents useful in this invention. The general procedures for obtaining a messenger RNA (mRNA) mixture is to prepare an extract from a tissue sample or to culture cells producing the desired protein, and to extract the mRNA by a process such as that disclosed by Cirgwin, et al., Biochemistry, 18:5294 (1979). The mRNA is enriched by poly(A) mRNA-containing material by chromatography on oligo (dT) cellulose of poly(U) Sepharose, followed by elution of the poly(A) containing mRNA-enriched fraction.

The above poly(A) containing mRNA-enriched fraction is used to synthesize a single-strand complementary cDNA (ss-cDNA) using reverse transcriptase. As a consequence of DNA synthesis, a hairpin loop is formed at the 3′ end of the DNA which will initiate second-strand DNA synthesis. Under appropriate conditions, this hairpin loop is used to effect synthesis of the second strand in the presence of DNA polymerase and nucleotide triphosphates.

The resultant double-strand cDNA (ds-cDNA) is inserted into the expression vector by any one of many known techniques. In general, methods, etc., can be found in Maniatis, supra, and Methods In Enzymology, Vols. 65 and 68 (1980); and Vols. 100 and 101 (1983). In general, the vector is linearized by at least one restriction endonuclease, which will produce at least two blunt or cohesive ends. The ds-cDNA is ligated with or joined to the vector insertion site.

If prokaryotic cells or other cells which contain substantial cell wall material are employed, the most common method of transformation with the expression vector is calcium chloride pretreatment as described by Cohen, R.N., et al., Proc. Nat'l. Sci. USA, 69:2110 (1972). If cells without cell wall barriers are used as host cells, transfection is carried out by the calcium phosphate precipitation method described by Graham and Van der Eb, Virology, 62:456 (1973). Other methods for introducing DNA into cells such as nuclear injection or protoplast fusion, have also been successfully used. The organisms are then cultured on selective media and proteins for which the expression vector encodes are produced.

Clones containing part or the entire cDNA for Factor VIII-C analogs are identified with specific antibodies directed against part or all of the Factor VIII-C analog protein. This method of identification requires that the ds-cDNA be inserted into a vector containing appropriate regulatory nucleic acid sequences adjacent to the insertion site. These regulatory sequences initiate transcription and translation of those ds-cDNA molecules inserted in the vector. Those clones containing Factor VIII-C analog cDNA sequences correctly positioned relative to the regulatory sequences, synthesize part or all of the Factor VIII-C analog protein. Such clones are detected using appropriately specific antibodies. Such a cloning system is the lambda gt11 system first described by Young and Davis, supra.

Clones containing the entire sequence of Factor VIII-C analogs are identified using the cDNA insert of the Factor VIII-C analogs recombinants isolated during the initial screening of the recombinant lambda gt11 cDNA library as a probe. Nucleotide sequencing techniques are used to determine the sequence of amino acids encoded by the cDNA fragments. This information may be used to determine the identity of cDNA clones as specific for human Factor VIII-C analogs by comparison to the known amino acid sequence of the amino-terminus of Factor VIII-C (Gitshier et al., and Wood et al., supra). Alternatively, identification may be confirmed by employing techniques such as Northern blot analysis and hybrid-selected translation or by comparison to Factor VIII-C clones isolated from other species, e.g., mouse, rat, etc.


## DETAILED DESCRIPTION OF THE DRAWINGS

### Figure 4

### Design of FVIII cDNA Variant (delta FVIII)

#### A. FVIII Protein:

The top figure depicts the complete FVIII protein molecule with the various A, B and C domains. The lower figure depicts the engineered truncated delta FVIII protein molecule. The B domain is devoid of amino acid residues 747-1560. The thrombin cleavage sites delineating the B domain (740/741 and 1689/1690) remain intact.

B. FVIII cDNA:

The top line illustrates the complete FVIII cDNA starting at the methionine initiation codon (ATG) and ending with the translation termination codon (TGA). The restriction sites EcoR1, Sph1 and BamH1 indicate the positions used to engineer the deleted (delta FVIII) cDNA.

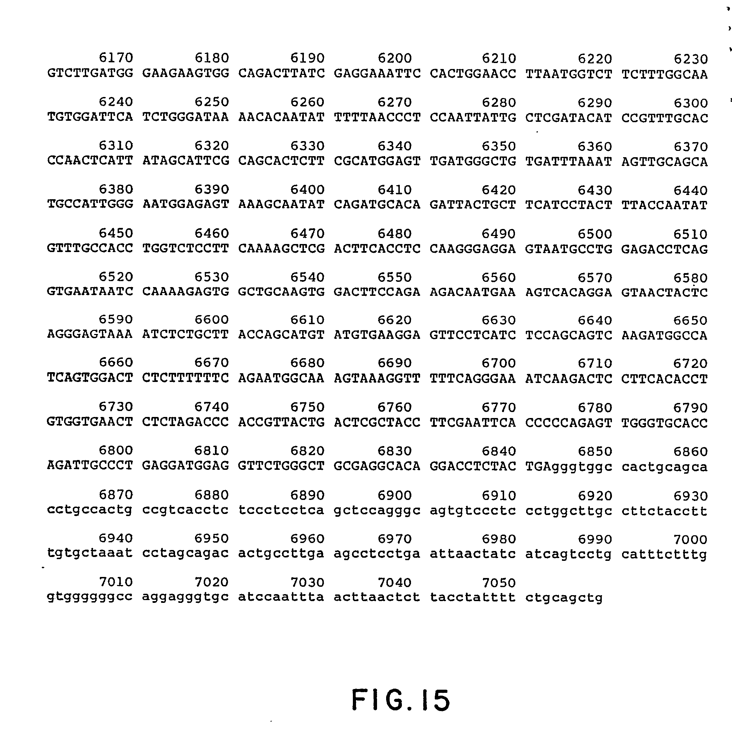

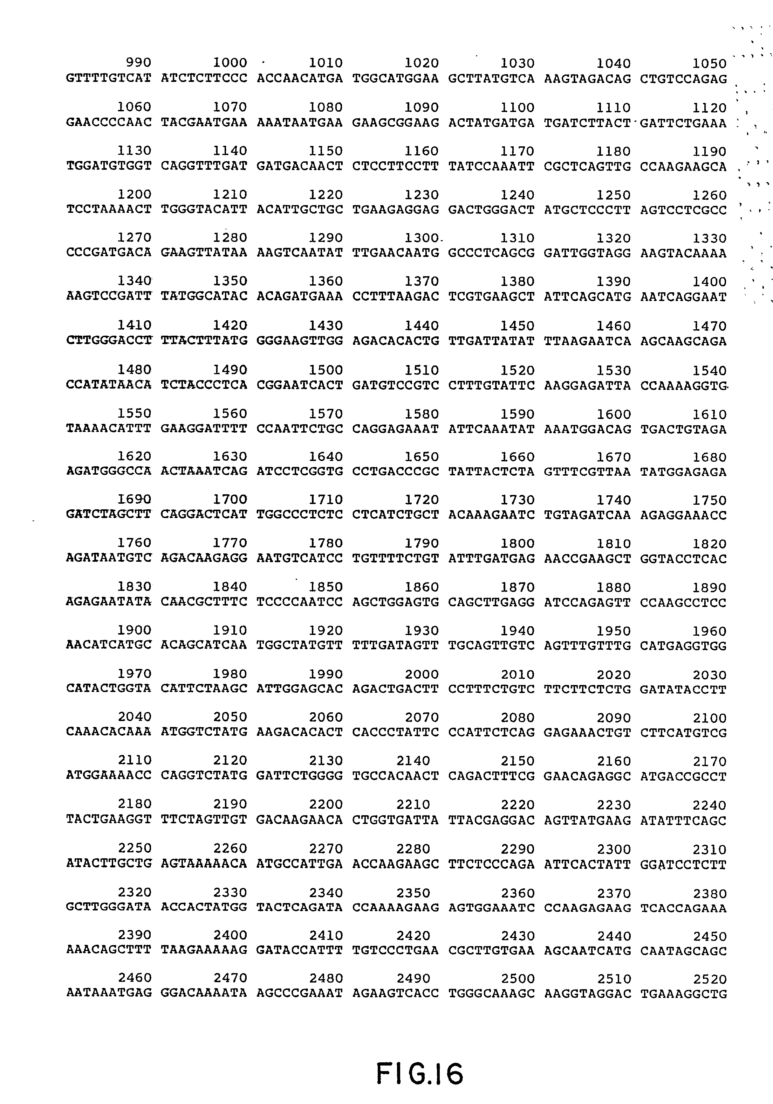

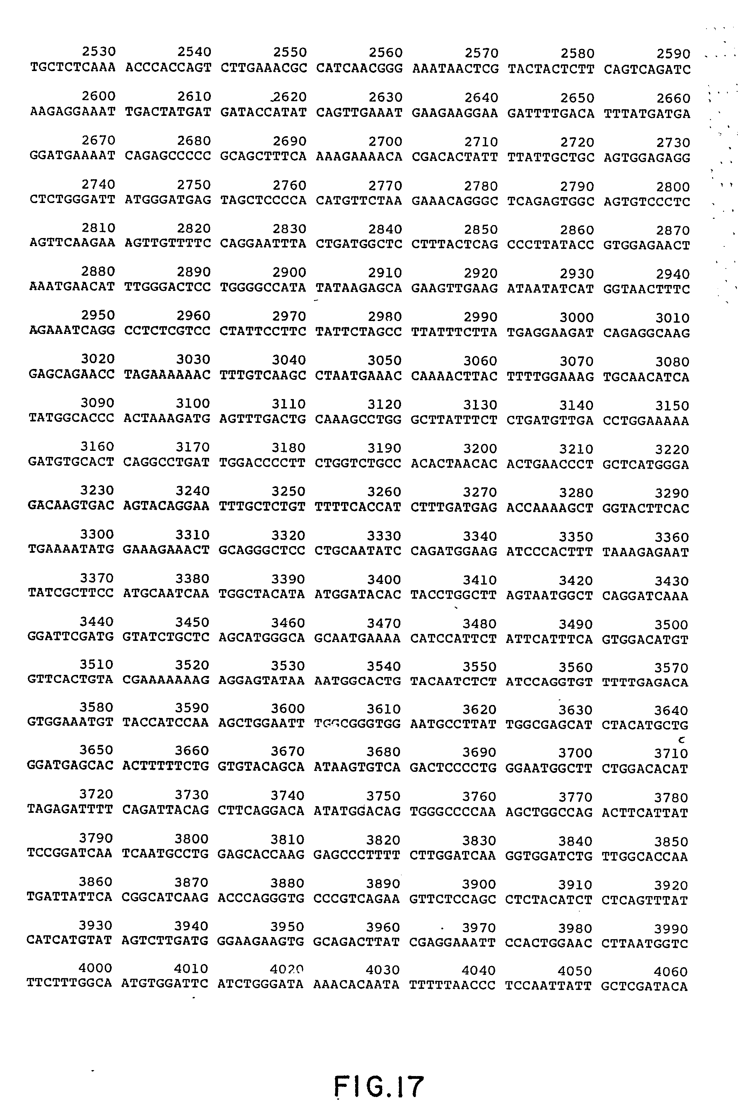

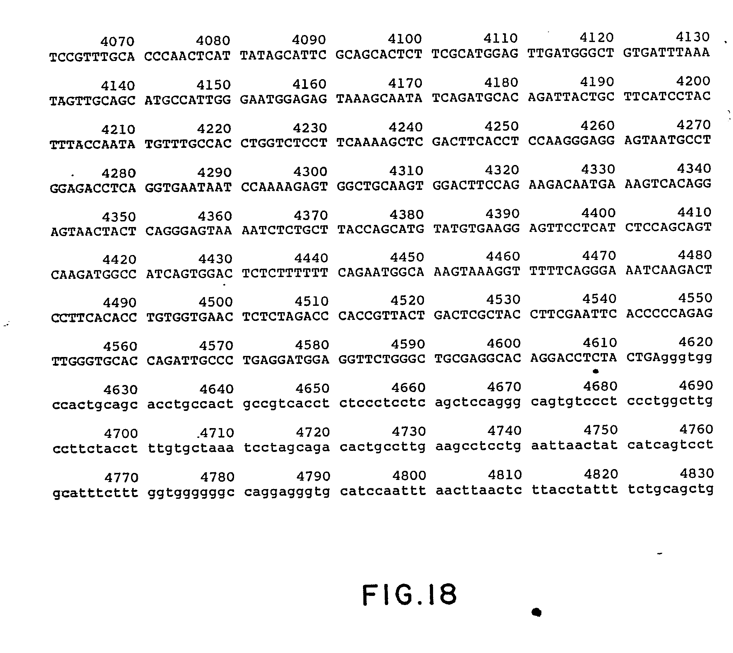

The lower figure illustrates the resulting variant. The deleted variant (delta FVIII) contains an internal, contiguous deletion, spanning nt 2296-4737 (see Figure 15-18).

Figure 23

A cDNA library was constructed in lambda gt11 from adult liver polyA-selected RNA (Ricca, unpublished) and a series of overlapping clones encompassing all but - 500 bp of EX14 region were identified and sequenced. A genomic clone of - 3000 bp spanning the missing region was isolated in EMBL3. This genomic clone and the appropriate cDNA clones were assembled into full length FVIII containing all 26 exons and cloned in pGEM2 (Promega). The resulting plasmid designated as p181-7. The following plasmids describe the construction schema used to generate a deletion in the exon 14 region of FVIII DNA. the numbering system used throughout is based on bp #1 being the A in the ATG translation initiation codon.

p159-1: This plasmid contains the entire 5' region (4744 bp) of FVIII plus additional 20 bp upstream of the ATG start codon. The 5' terminus has been modified to an XhoI recognition site with synthetic linkers (collaborative research) for subsequent cloning. The 3' end is deliminted by FVIII BamH1 site (nt 4744).

p167-2: p159-1 was cleaved to completion with Sph1 (nt 3936) followed by a partial digest with EcoR1 (nt 2290). The ensuing gap was bridged with the following synthetic oligonucleotide containing cohesive EcoR1 and Sph1 ends (5-3) and an internal BamH1 site:

<u>EcoR1</u>          <u>BamH1</u>     <u>Sph1</u>

AATTCACTATTGGATCCTGCGGCATG

GTGATAACCTAGGACGCC

The sequence between the EcoR1 to the BamH1 sites is identical to nucleotides 2290-2295 followed by nucleotides 4738-4744 in FVIII map.

p182-4: A plasmid containing the fully assembled FVIII cDNA (p181-7) was cleaved with BamH1 and the resulting large fragment, devoid of the internal BamH1 fragment (nt 1870-4744), was doubly purified electrophoretically in agarose gel and recovered by electroelution. Plasmid p167-x was cleaved with BamH1, the small internal fragment containing the deletion in exon 14 similarly purified, and then ligated to the large BamH1 fragment of p181-7. DNA was used to transform competent E. coli HB101 and plasmid DNA isolated from several ampicillin resistant transformants analyzed by restriction mapping to identify plasmids harboring the mutated DNA with the BamH1 fragment in the proper orientation. Plasmid DNA was purified and the sequence across the mutated region verified by Maxam Gilbert sequencing. The salient features of p182-x include a 2442 bp internal deletion in exon 14 (nt 2296-4737); 20 bp upstream and 214 bp downstream of FVIIIC coding region, and an XhoI site at the 5' and 3' termini.

Figure 24

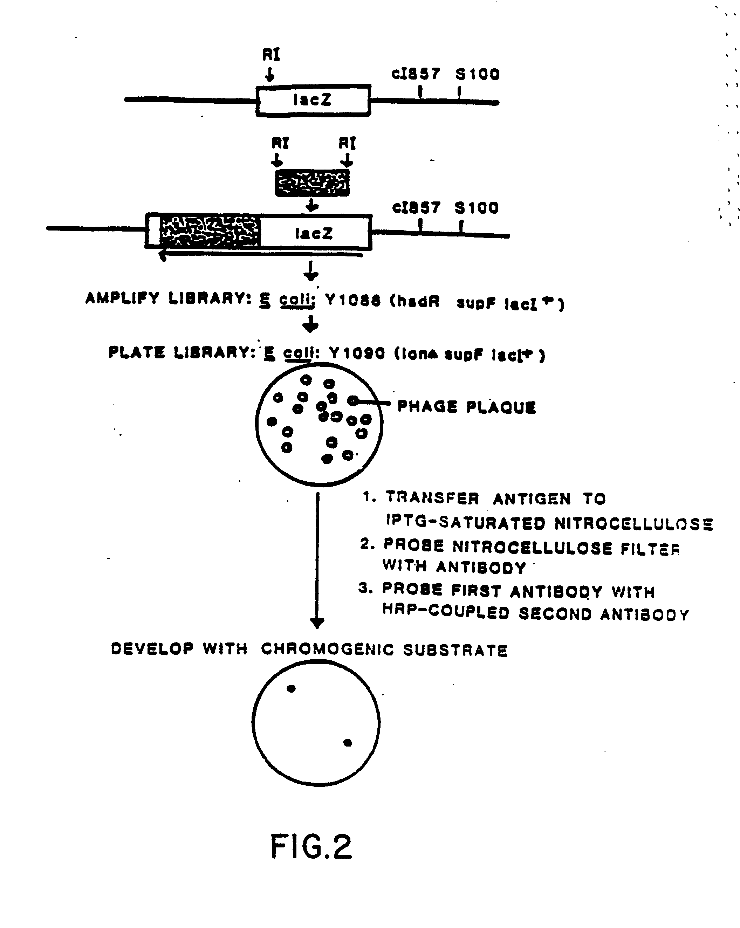

p182-4-ATG-C: To increase translation efficiency, the region surrounding the ATC codon was replaced with synthetic oligonucleotide conforming to a consensus 'AUG' sequence (Kozak 1984) but otherwise identical to FVIII sequence. This was accomplished by cleaving p182-4 at the AatII site located in the pGEM2 sequence upstream of FVIII insert, and Sac1 (nt 15, Figure 2). The large fragment lacking the AstII/Sac1 fragment was purified and ligated to the synthetic oligonucleotide indicated below containing an internal consensus ATG sequence, cohesive AatII and Sac1 termini (5-3) and internal XhoI site 20 bp upstream of the ATG codon

|   AatII | XHO1 | Consensus | SAC1 |
|---|---|---|---|

CCAAGCTTCTCGAGAACATTTGTAGCAATCCACCATGGGAATAGAGCT

TGCAGGTTCGAAGAGCTCTTGTAAACATGGTTAGGTGGTACCCTTATC

Figure 25

p-261-26: delta FVIII insert was recovered as an XHO1 fragment from p182-4-ATG-C and cloned in BPV shuttle vector p143-7. The resulting recombinant vector, p261-26, contains the following genetic elements described in clockwise direction: BPV genomic lacking 1485 bp frm its nontransforming region (nt 5473-6958 in BPV map, Chen, 1982); PML2d sequence, Moloney murine sarcoma virus (MSV) enhancer; followed by mouse metallothionein promotor (both described in Sarver, et al., 1985); delta FVIII DNA insert from p182-ATG-C; and RNA processing signals from SV40 genome (described in Sarver, et al., 1985).

p188-3: This expression vector is identical to p261-26 except for the presence of a full-length FVIII cDNA insert (from p182-4) instead of the deleted version.

## Table 1

### Coagulation Activity Produced in Clonal Isolates Harboring Delta FVIII (p261-26)

| Cell Line | u/ml |
|---|---|
| NS261-49 | 0.93 |
| NS261-50 | 0.99 |
| NS261-118 | 1.325 |
| NS261-78 | 0.850 |
| NS261-131 | 1.085 |

Supernatant media from arbitrarily selected transformed lines were assayed for delta FVIII biological activity defined as activity which will correct the coagulation defect of FVIII deficient plasma. The activity is expressed as the clotting time required for the formation of a visible fibrin clot in Factor VIII deficient plasma upon the addition of samples containing Factor VIII activity. The shorter the clotting time, the greater the Factor VIII activity in a given sample. When used in conjunction with reference samples with known activity levels (U/ml), the clotting time is then proportioned to Factor VIIIC activity level. As shown above, coagulation activities in these isolates the range from 0.93-1.325 u/ml of media. Normal levels of circulating FVIII are defined as 1 u/ml.

The recombinant delta FVIII protein was also known to be neutralizable by monoclonal antibody directed against the 72 kd carboxy subunit of the protein, to be inhibited by EDTA (SmM) due to chelation of $CA^{+2}$ ions which are necessary for the association of the various peptides in the active molecules, and to be activated by thrombin (ten-fold increase in activity within one minute of incubation of 37°C).

Protocol for Coagulation Assay

## 1. Preparation of Reagents :

All assays were performed in 12 x 75 polystyrene tubes (American Scientific Products). Factor VIIIC deficient plasma (George King Biomedical, Inc., Overland Park, KS) was removed from -70°C freezer, thawed at 37°C and transferred to 12 x 75 polystyrene tube. Actin (activated cephaloplastin, stored at 4°C) was transferred to 12 x 75 tube and placed on ice until use. A solution of 35 mM calcium chloride was prewarmed at 37°C. Reference normal plasma (George King Biomedicals) was thawed or reconstituted as per supplier and placed on ice until use.

## 2. Protocol:

Immediately before use, an appropriate dilution of the reference was made. in citrate albumin buffer (CAB, 14.3 mM Na citrate/12.8 mM NaCl/6.1 mM Na Azide/0.5% albumin, pH 6.7) resulting in a 1 U/ml Factor VIIIC concentration. Additional serial dilution at 1:20, 1:40, 1:80 and 1:160 were made in CAB solution. To a cuvette which was been preheated at 37°C and in rapid succession, were added 100 mu l of Factor VIIIC deficient plasma, 100 mu l of actin, and 100 mu l of 1:20 dilution of the reference. The procedure was repeated for the remaining dilutions at 90-second intervals.

Each mixture was incubated for precisely 5 minutes. The coagulation cascade was initiated with the addition of 100 mu l of prewarmed calcium chloride (35mM), and the time required for the formation of a fibrin clot was carefully monitored.

For analysis of test sample containing greater than 0.4 U/ml, the same dilution as for the reference was used. Samples containing less than 0.4 U/ml a 2-fold dilution of series beginning with 1:5 was used. All samples were handled as described above.

## 3. Calculations:

A standard curve is obtained by relating clotting times to Factor VIIIC activity in reference samples. Clotting times at each dilution are plotted on the ordinate of a log-log paper and the reciprocol of the dilution is plotted on the abscissa. A test fit line is drawn. Results obtained with the samples are drawn on same graph and a line of best fit which also parallels the reference line is constructed. The ratio between dilutions for the reference and the samples which give the same clotting time is determined. This ratio is then multiplied by the assigned activity of the reference to obtain activity/ml in the sample.

## EXAMPLE

### A. Preparation of Total RNA

Total RNA (messenger, ribosomal, and transfer) was extracted from fresh human umbilical vein endothelial cells essentially as described by Chirgwin, supra, (1979). Cell pellets were homogenized in 15 volumes of a solution containing 4 M guanidine thiocyanate, 25 mM sodium citrate at pH 7.0, 0.5% N-laurylsarcosine, 0.1 M 2-mercaptoethanol, and 0.2% Antifoam A (Sigma Chemical Co., St. Louis, MO). The homogenate was centrigued at 6,000 rpm in a Sorvall GSA rotor for 15 minutes at 10°C. The supernatant fluid was adjusted to pH 5.0 by addition of acetic acid and the RNA precipitated by 0.75 volumes of ethanol at -20°C for two hours. RNA was collected by centrifugation and dissolved in 7.5 M guanidine hydrochloride containing 2 mM sodium citrate and 5 mM dithiotreitol. Following two additional precipitations using 0.5 volumes of ethanol, the residual guanidine hydrochloride was extracted from the precipitate with absolute ethanol. RNA was dissolved in sterile water, insolvable material was removed by centrifugation, and the pellet was re-extracted with water. The RNA was adjusted to 0.2 M potassium acetate and precipitated by addition of 2.5 volumes of ethanol at -20°C overnight.

## B. Preparation of Poly(A)-containing RNA

The total RNA precipitate, prepared as described above, was dissolved at a concentration of 40 $A_{260}$ units in 20 mM HEPES buffer (pH 7.2) containing 10 mM EDTA and 1% SDS, heated at 65°C for 10 minutes, then quickly cooled to 25°C. The RNA solution was then diluted with an equal volume of water and NaCl was added to bring the final concentration to 300 mM NaCl. Samples containing up to 24000 $A_{260}$ units of RNA were chromatographed on poly(U)-Sepharose using standard procedures. Poly(A)-containing RNA was eluted with 90% formamide containing 1 mM HEPES buffer (pH 7.2), and 2 mM EDTA. The eluate was adjusted to 0.24 M NaCL, and the RNA was precipitated by 2.5 volumes of ethanol at 02-°C.

## C. Construction of cDNA Clones in Lambda gt₁₁

The procedures followed for the enzymatic reaction is shown in Figure 1. The mRNA (20 ug) was copied into ds-cDNA with reverse transcriptase and DNA polymerase I exactly as described by Buell, et al., supra, and Wildensen, et al., J. Biol. Chem., 253 :2483 (1978). The ds-cDNA was desalted on Sephadex G-50 and the void volume fractions further purified on an Elutip-D column (Schleicher & Schuell, Kenne, NH) following the manufacturer's directions. The ds-cDNA was made blunt-ended by incubation with S1 nuclease, [Ricca, et al., J. Biol. Chem. 256:10362 (1981)]. The reaction mixture consisted of 0.2 M sodium acetate (pH 4.5), 0.4 (M) sodium chloride, 2.5 mM zinc acetate and 0.1 unit of S1 nuclease per ng of ds-cDNA made to a final reaction volume of 100 ul. The ds-cDNA was incubated at 37°C for one hour, extracted with phenol:chloroform, and then desalted on a Sephadex G-50 column as described above .

The ds-cDNA was then treated with EcoRI methylase and DNA polymerase I (Klenow) using reaction conditions described in Maniatis (supra). The cDNA was again desalted on a Sephadex G-50 as decribed above and then ligated to 0.5 g of phosphorylated EcoRI linkers using T₄ DNA ligase (Manitis, supra). DNA with a size greater than 1 kilobase was eluted from the gel, recovered by binding to an Elutip-D column, eluted with 1 M Nacl and then collected by ethanol precipitation.

As shown in Figure 2, the DNA fragments were then inserted into Eco RI-cleaved and phosphatase-treated lambda gt₁₁ using T₄ DNA ligase to produce a library of approximately twelve (12) million phage of which 50% contain inserts (i.e., six million clear plaques on x-gal plates). The library was amplified by producing plate stocks at 42°C on E. coli Y1088 [supE supF metB trpR hsdR⁻ hsdm⁺ tonA21 strA lacU169 proc::Tn5 (pMC9)]. Amplification procedures are described in Maniatis, (supra). Important features of this strain described by Young and Davis, include (1) supF (required suppression of the phage amber mutation in the S gene), (2) hsdR⁻ hsdm⁺ (necessary to prevent restriction of foreign DNA prior to host modification), (3) lacU169 (a deletion of the lac operon which reduces host-phage recombination and which is necessary to distinguish between lambda gt₁₁ recombinants and non-recombinants), and (4) pMC9 (a lacI-bearing pBR322 derivative which represses, in the absence of an inducer, the expression of foreign genes that may be detrimental to phage and/or cell growth). The tier of the amplified library was determined to be $8 \times 10^{10}$ pfu/ml. Albumin cDNA clones represent 3-5% of the clear plagues in the library as judged by hybridization with a nick-translated albumin cDNA clone.

## D. Identification of Clones Containing Factor VIII-C Sequence

To screen the library for Factor VIII-C antigenic determinants producing clones, $1.1 \times 10^7$ lambda gt₁₁ recombinant phage were plated (70,000 pfu/plage, 160 plates) on a lawn of E. coli Yo1090 [lacU169 proA⁺ lon araD139 strA supF [trpC22::Tn10] (pMC9)] and incubated at 42°C for 3 hours. This host is deficient in the lon protease, thereby reducing the degradation of expressed foreign proteih. Nitrocellulose filters, previously saturated with 10 mM isopropyl thio-B-d-galactopyranoside (1PTG) and dried, were overlaid on the plates. The plates were then removed to 37°C incubator overnight. Since IPTG is an inducer of lacZ transcription, the expression of foreign DNA inserts in lambda gt₁₁ is under common control with lacZ transcription and, as such, is also induced. The position of the filters was marked with a needle, the filters removed, washed in TBS buffer (20 mM Tris, pH 7.5 and 500 mM NaCl), and incubated in TBS plus 3% gelatin for 30 minutes at room temperature.

The filters were then incubated at room temperature overnight in a 1:100 dilution of an emu polyclonal antibody directed against Factor VIII-C in a buffer consisting of 1% gelatin in TBS. The antibody was prepared as described by Fulcher and Zimmerman, Proc. Nat. Acad . Sci. USA, 79:1648 (1984).

After 2 thirty-minute washes with TBS at room temperature, the filters were then incubated with a 1:200 dilution of rabbit anti-emu IgG. After 2 additional 30 minute washes with TBS, the filters were incubated with a 1:2,000 dilution of horseradish peroxidase (HRP) conjugated goat anti-rabbit antisera (Bio-Rad, Richmond, CA). The filters were then incubated for 2 hours at room temperature, and washed twice for 30 minutes in TBS. The filters were incubated at room temperature in HRP color development solution as described in the Bio-Rad accompanying literature. Twenty-six putative positive plaques were identified.

A 4-mm diameter plug at the position of the color development signal was removed from the plates and incubated in 10 mM Tris HCl, pH 7.5, and 10 mM MgSO₄ overnight. Approximately $10^3$ plaque-forming units (PFU) were replated on 90 mm plates and rescreened as described above. Five clones remained positive through the second cycle of screening. This replating and rescreening process was repeated until all plaques on the plate produced a signal.

Phage DNA was isolated from each of the 5 clones. Restriction endonuclease cleavage analysis indicated that the 5 clones were identical and probably resulted as an artifact of the amplification procedure.

The cDNA insert from one of the clones was excised using a combination of the restriction enzymes EcoRI and PvuI. The EcoRI site to the right of the cloned cDNA was unexpectedly missing in all 5 clones; thus PvuI, which cuts at position approximately 20066 Kb in lambda gt₁₁, was used in combination with EcoRI. The cDNA insert (approximately 1.2 k:lobases in length) was subcloned between the PvuI and EcoRI sites of pSP64 and subsequently used as a probe to rescreen the lambda gt₁₁ library and a human placental genomic library constructed in bacteriophage EMBL3.

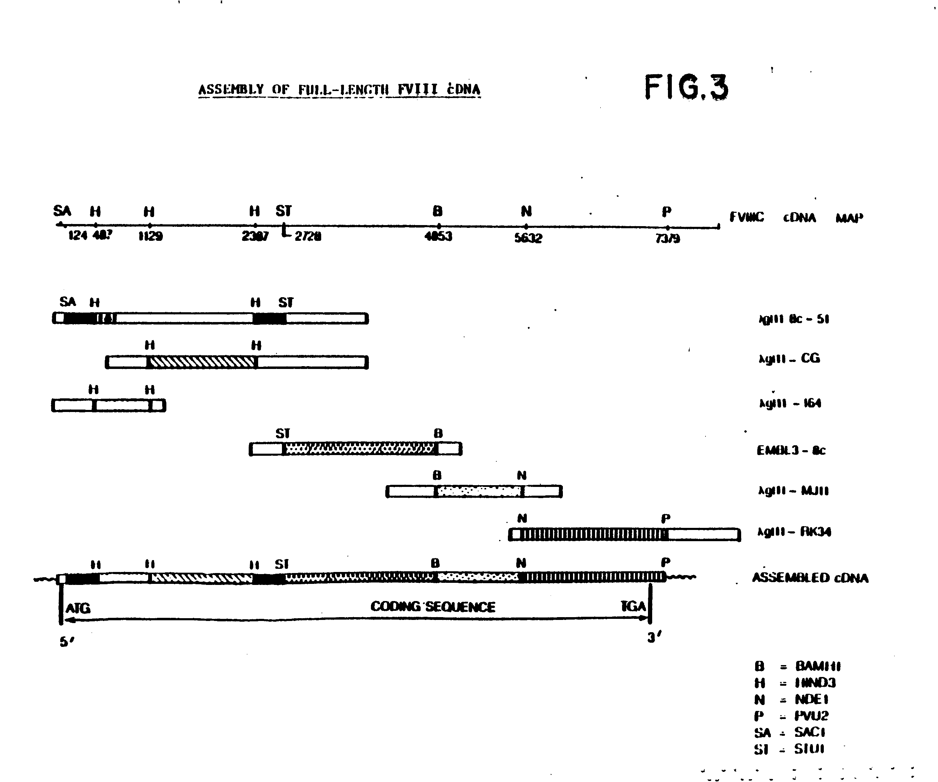

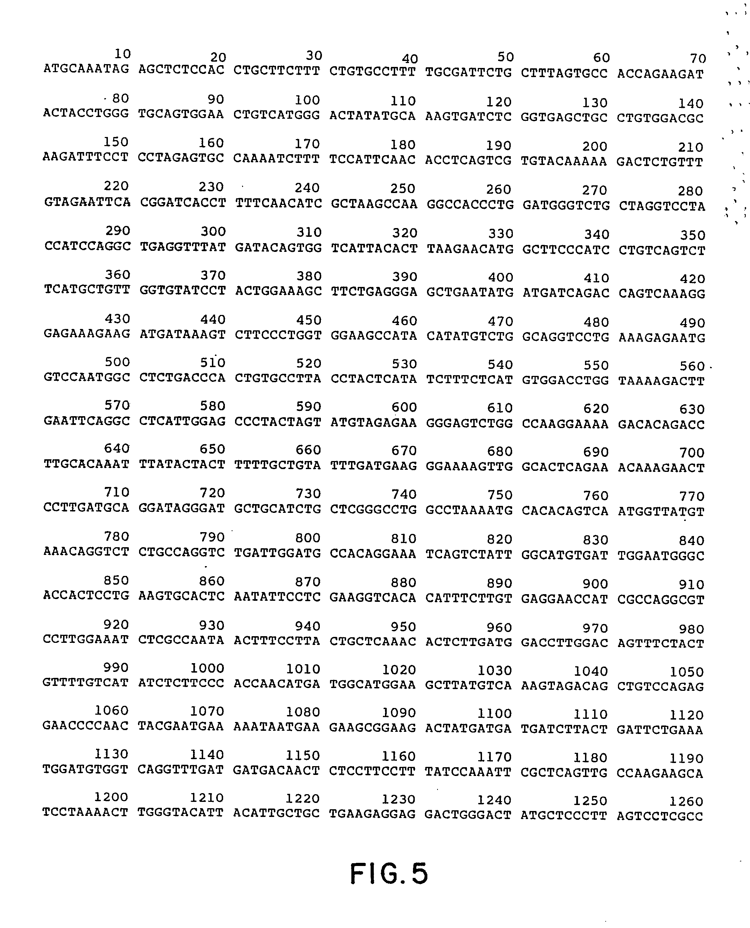

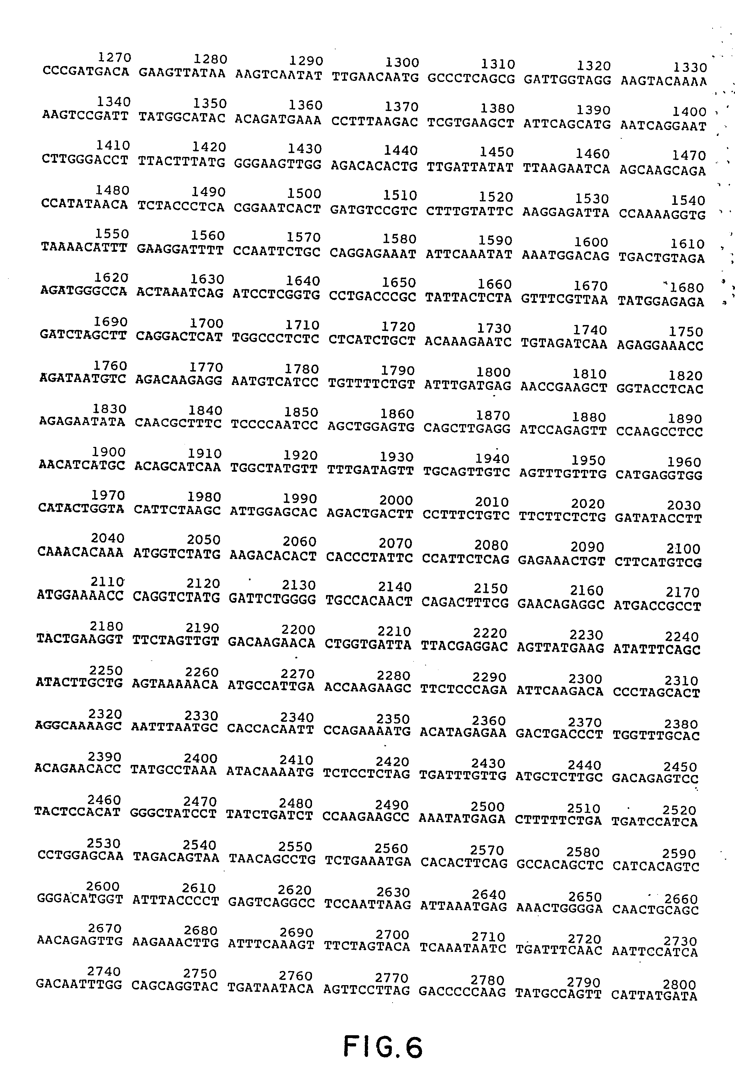

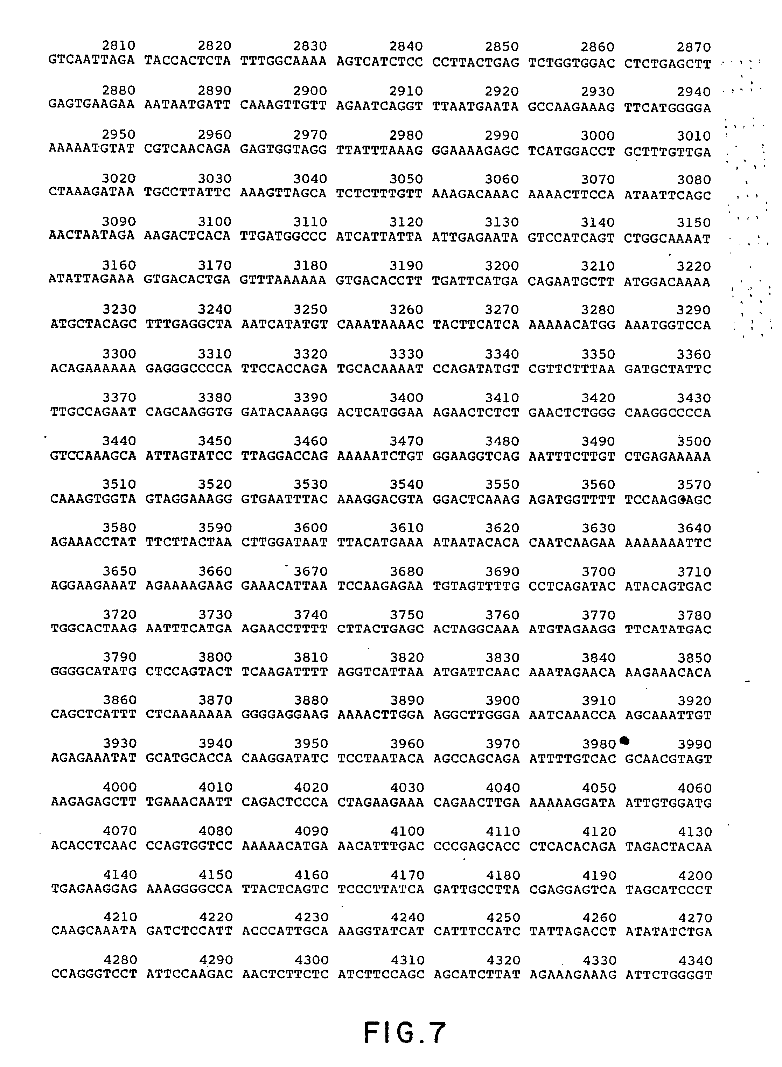

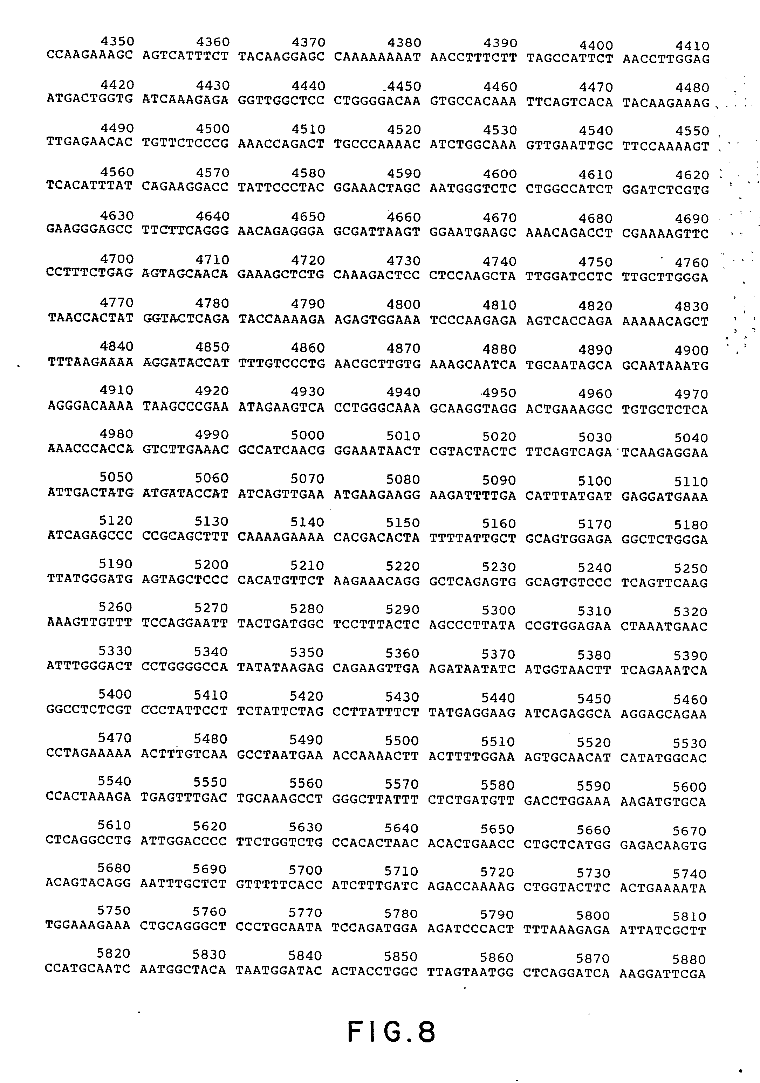

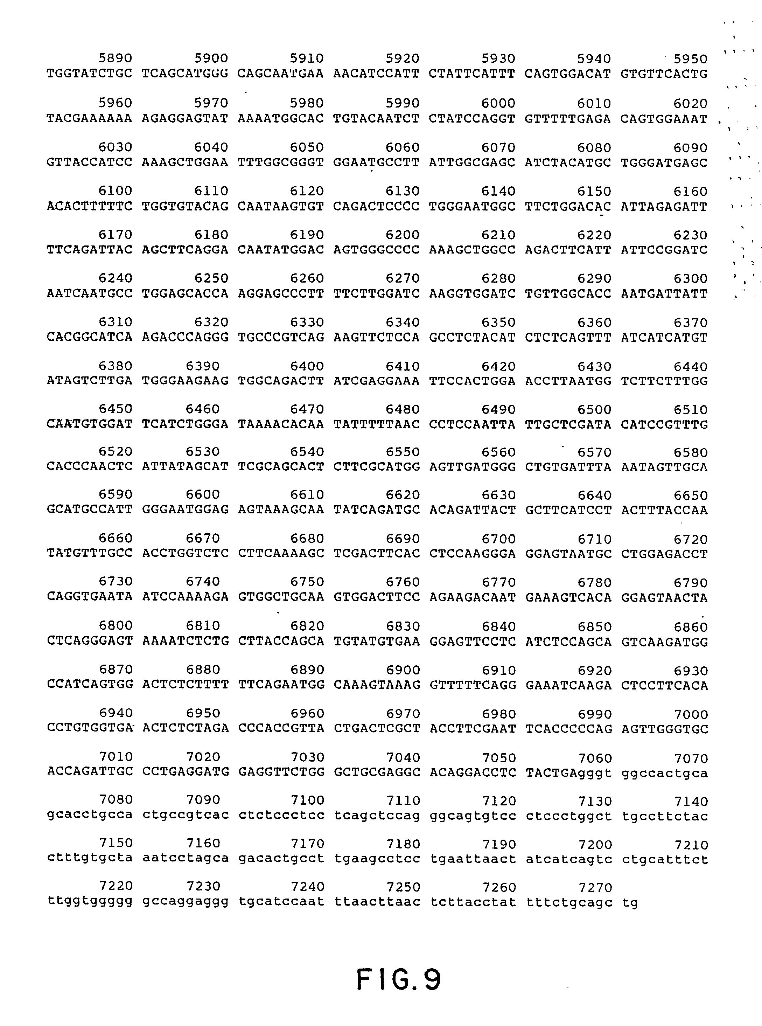

A series of overlapping clones extending to the 5′ and 3′ direction of the original antibody positive clones and encompassing full-length Factor VIII-C were thus identified. The complete set of overlapping clones was sequenced and then assembled into a full-length cDNA clone as indicated diagrammatically in Figure 3. The DNA sequence of the full-length Factor VIII-C coding region as assembled is given in Figures 5-9. Our designation for this clone is pd delta BPV/F8C-20 (p188-3).

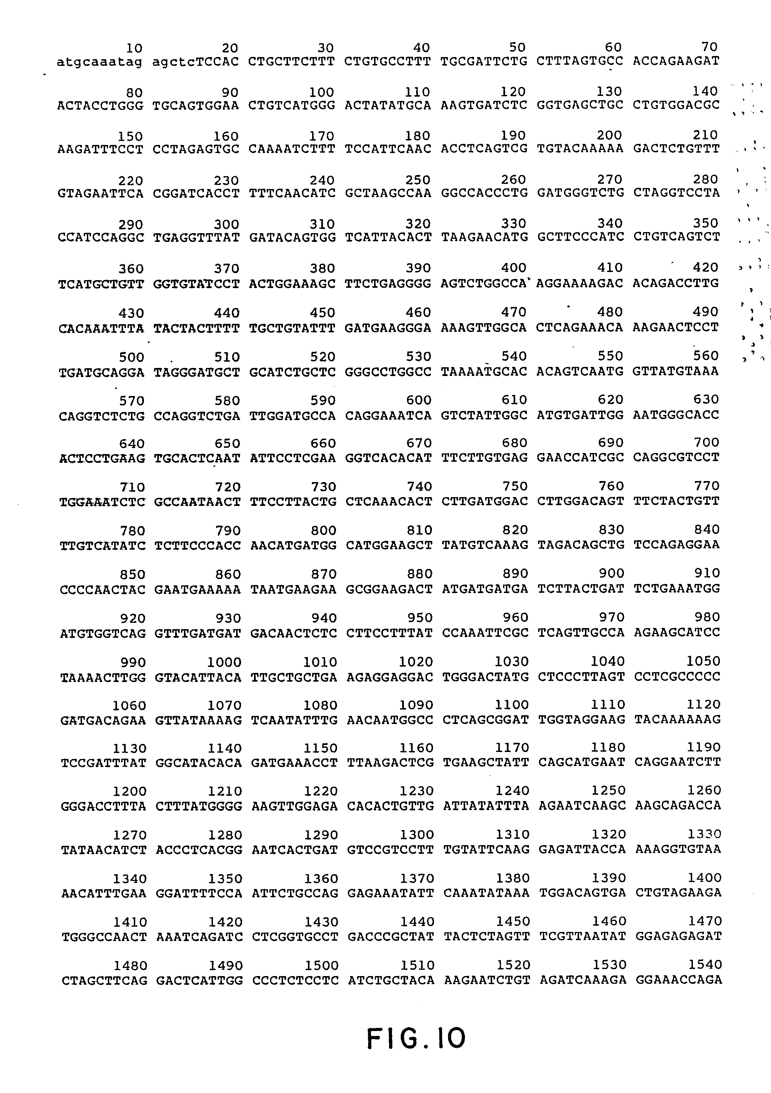

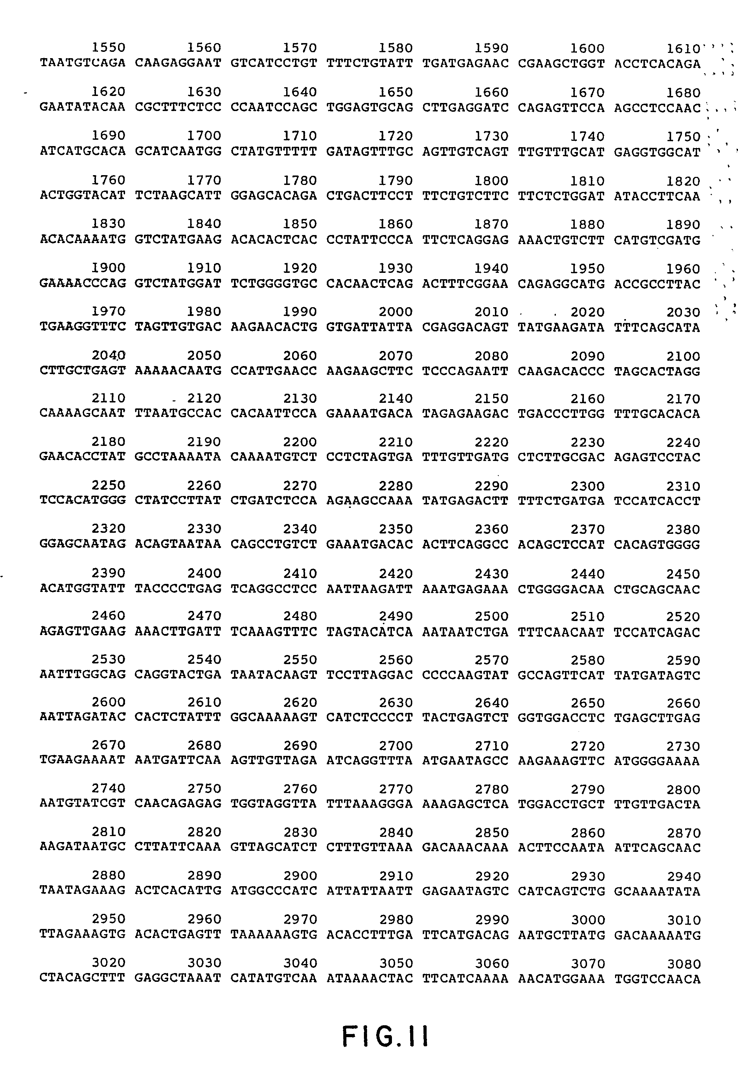

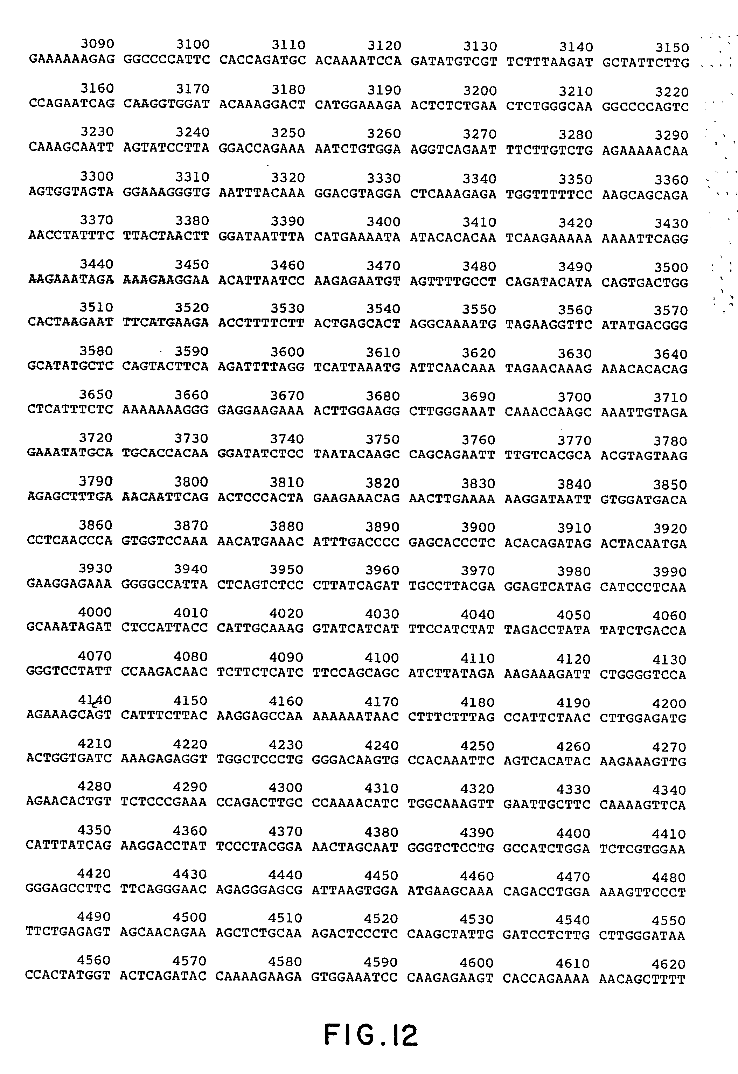

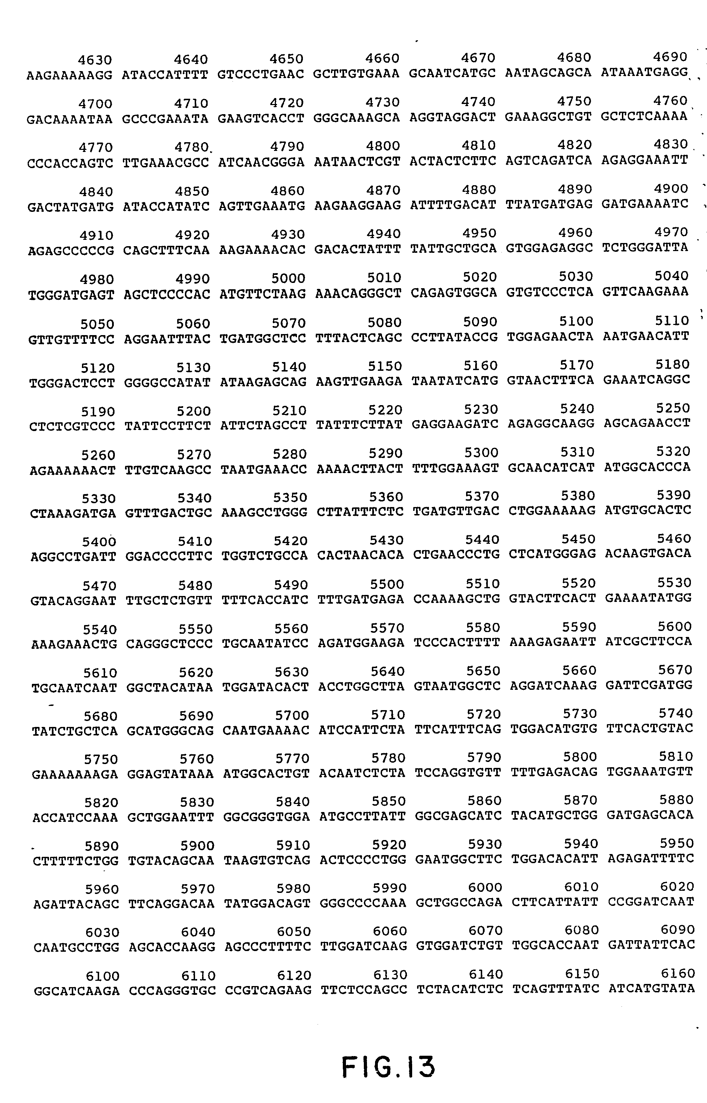

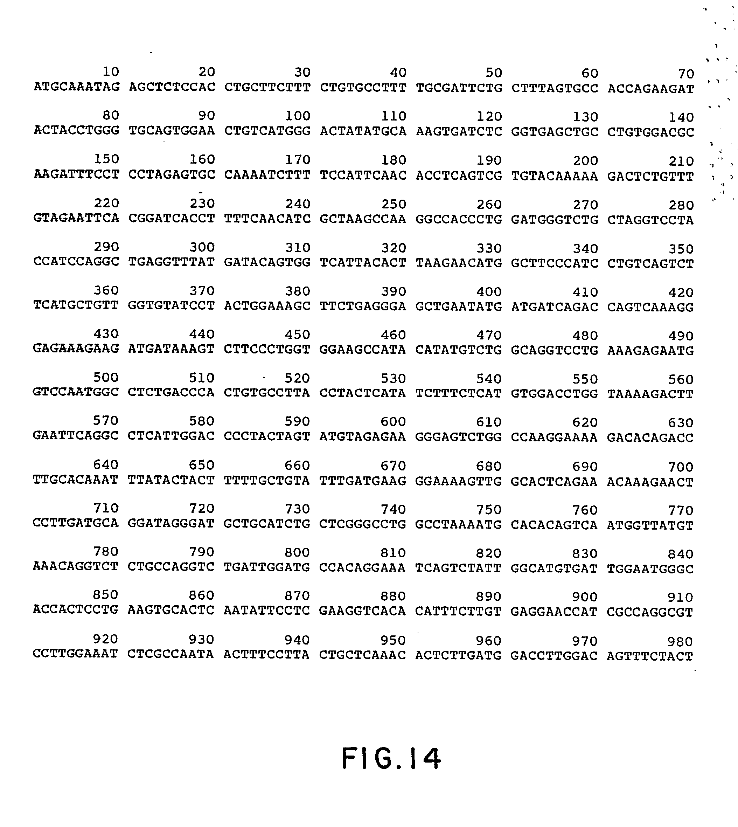

During the assembly of the full-length Factor VIII-C sequence, one cDNA clone was identified in which the nucleotides comprising the entire Exon 4 (nucleotides 389-601 in Gitschier, et al., supra, were missing. The deletion mapped in the Factor VIII-C analog is exact and complete in that it consists of 213 base pairs, all of which comprise Exon 4. The deletion does not overlap into any other Exon and is not a subset of Exon 4. Additionally, the 213 base pair deletion results in the removal of exactly 71 amino acids from the final Factor VIII-C protein. Thus, this deletion conserves the open reading frame of the cDNA in that the amino acid content and sequence of the remaining portion of the Factor VIII-C peptide is identical to native Factor VIII-C. The complete DNA sequence of this Factor VIII-C analog (pd BPV/F8C-20-Ex4) is shown in Figures 10-14.

Two sources of the Exon 4 deletion discovered in this cDNA are possible: (1) an in vitro artifactual cloning event, or (2) a naturally occurring mRNA from which this cDNA has been transcribed. Artifactual deletions are random, variable in size, and non-conservative. At best, they result in a mutant protein of similar biological function as the native protein. Most often, however, they result in non-functional proteins or in premature termination of the translation process. The precise, complete, and conservative nature of this deletion argues that this cDNA is the reverse transcript of a naturally occurring mRNA which resulted from an alternate in vivo splicing of the Factor VIII-C RNA. Figures 10-14, therefore, depict what is believed to be a naturally occurring Factor VIII-C analog.

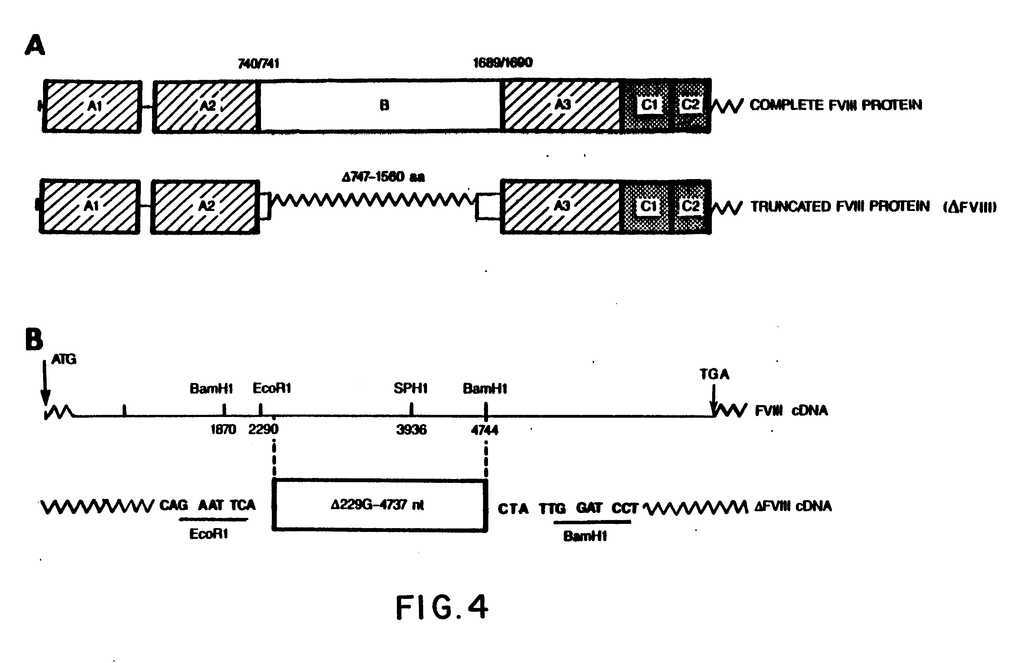

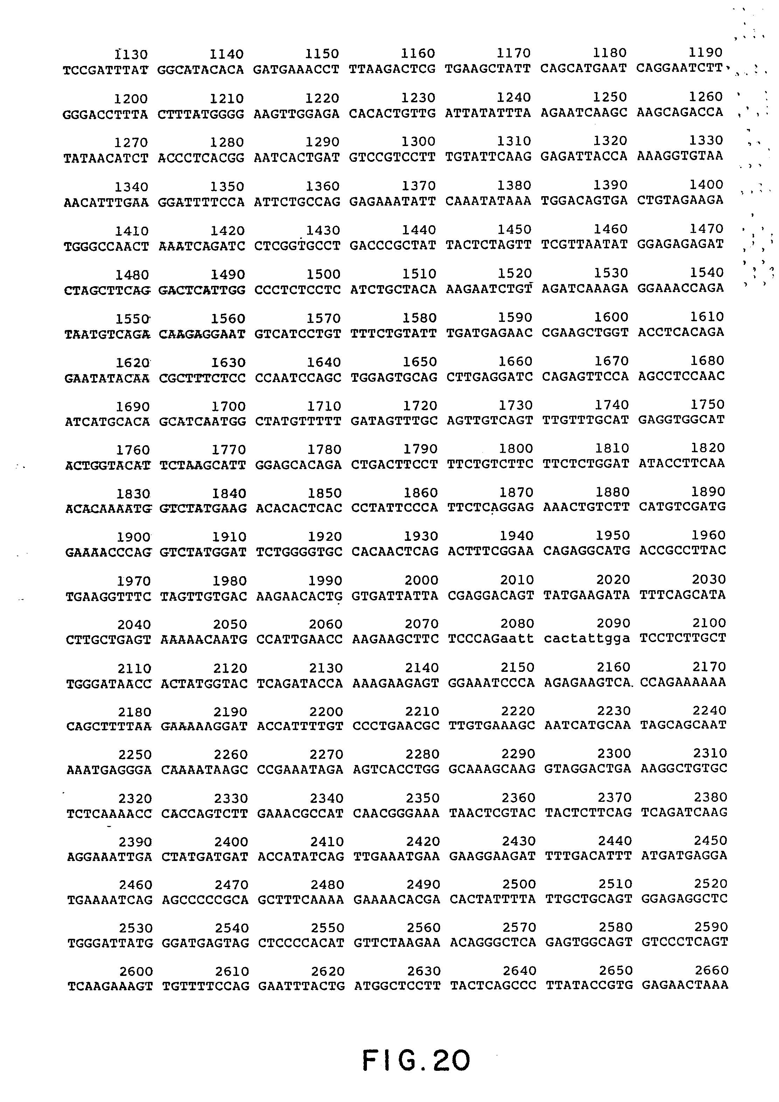

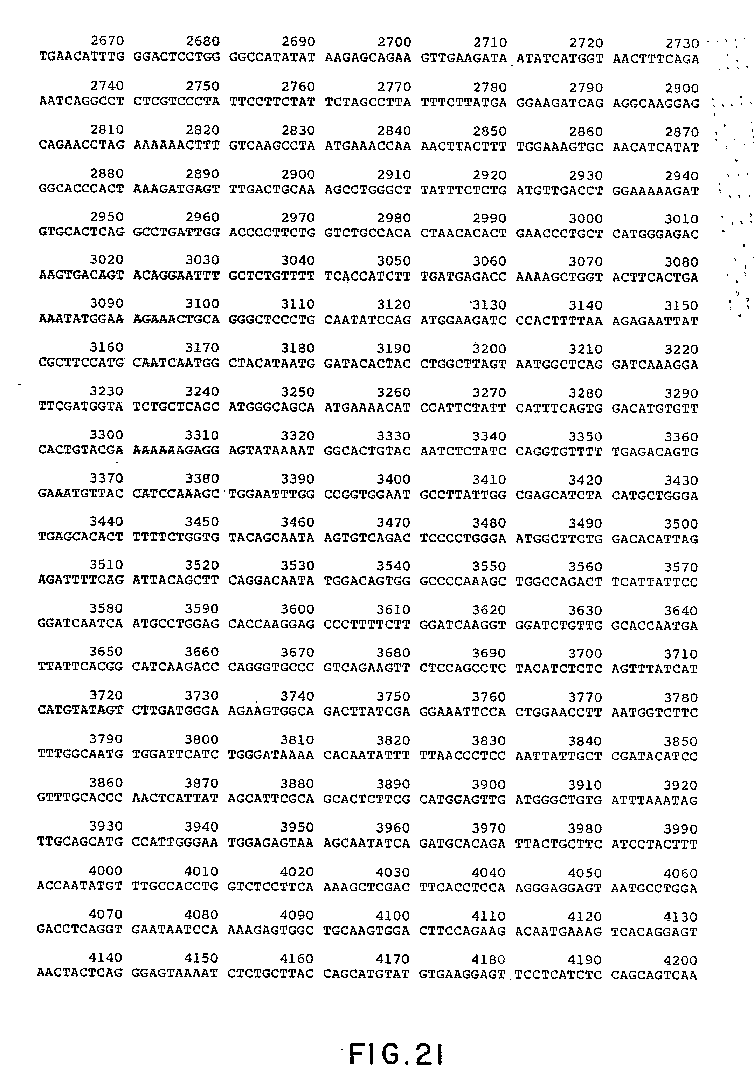

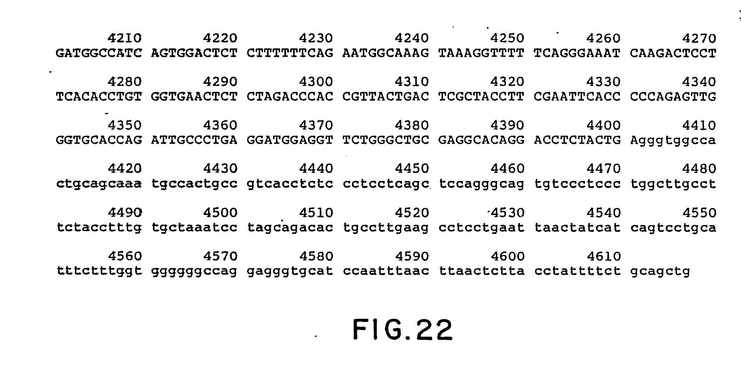

Other Factor VIII-C analogs have been genetically engineered. The sequence of two such analogs are shown in Figures 15-18 and Figures 12-22. As shown in Figures 4 and 15-18, a Factor VIII-C analog DNA has been engineered in which nucleotides 2296-4737 (as numbered in Figures 5-9) have been removed. This region contains a great portion of, but not all, Exon 14 deleted. The deletion is contained within the Exon 14 region and encodes a portion of the Factor VIII-C molecule which is removed during thrombin activation of Factor VIII-C during the clotting cascade. Our designation for this clone is pd delta BPV/delta F8C-ATG-C (26126).

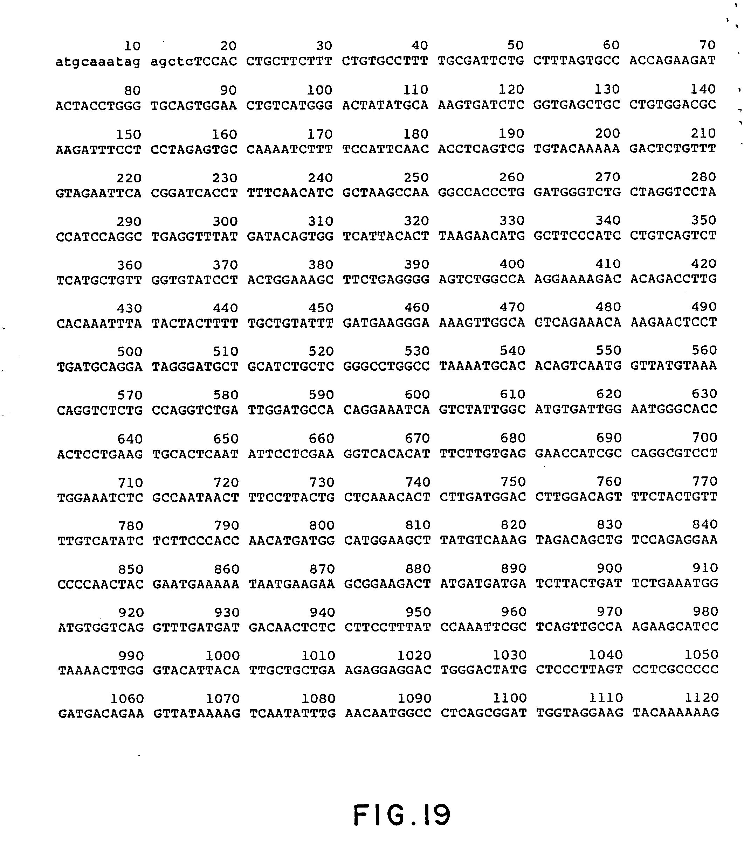

A second Factor VIII-C analog has been engineered in which the Exon 4 deletion and the engineered deletion of most of Exon 14 are combined, resulting in the cDNA whose sequence is shown in Figures 19-22.

## E. Construction of Expression Vector

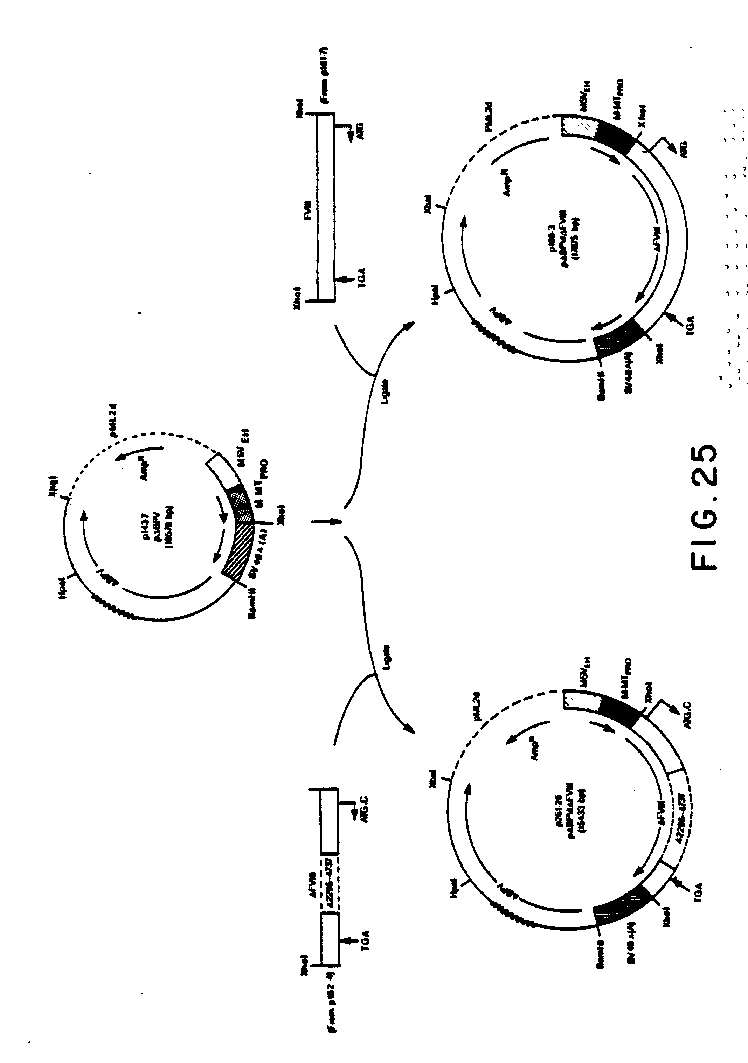

The assembled Factor VIII-C analog coding sequences shown in Figures 10-14, Figures 15-18 and Figures 19-22 were inserted into a bovine papillomavirus (BPV)-based shuttle vector constructed at Meloy Laboratories, Inc. A diagrammatic restriction map of the resulting pdBPV/Factor VIII-C analog recombinant DNA is illustrated in Figure 25. A detailed description of the generic elements in pdBPV/Factor VIII-C analog follows. The elements are described in a clockwise direction beginning with the 5' end of BPV DNA.

BPV DNA: The complete BPV genome (early and late regions) from which the sequence between nucleotides 5463 and 6958 had been deleted is contained in this construct. This sequence was shown to be dispensible for cellular transformation and extrachromosomal replication (Sarver, unpublished results). The HindIII recognition sequence at the site of deletion has been abolished.

pML2D1: This pBR322 derivate lacks the sequences (1095-2485) which inhibit replication of the prokaryotic DNA in mammalian cells [Lusky and Botchan, Nature 293:79 (1981)]. The original pML2 was further modified by deleting the sequences between the HindIII and SalI (29 and 651, respectively, in the pBR322 map). The original HindIII site was converted to a BamHI site, whereas the original SalI site was retained.

M-MSV Enhancer: The Moloney murine sarcoma virus enhancer fragment extends from the HinfI (Base 141) to the XbaI (base 525) site in the proviral long terminal repeat ([Dhar, et al., Proc. Natl. Acad. Sci. USA, 77:3937 (1980)]. Both sites were converted to BamHI sites with synthetic linkers to facilitate cloning.

M-MT Promoter: The mouse metallothionein promoter is the KpnI [approximately (-)300] to BglII [(+)-365] fragment with the CAP site (mRNA start site) at (+)301. The BglII site was converted to an XhoI site with synthetic linkers to facilitate cloning. Since the coding region of the M-MT gene beings downstream (at +374) from the 3' end (position +365) of the promoter described herein, no fusion products result.

Factor VIII-C Analog Insert: Derivation of the complete Factor VIII-C cDNA (sans most of the Exon 14 coding sequences) is detailed in Section D above. The insert contains 20 base pairs 5' upstream of the first ATG codon in the Factor VIII-C sequence, the sequence encoding for the signal peptide, the coding region, a translational termination codon, and 214 base pairs of Factor VIII-C 3' flanking sequences. The fragment terminates with a PvuII site. The 5' and 3' termini were modified to XhoI sites (with synthetic oligonucleotides and synthetic linkers, respectively) to facilitate subsequent cloning. This fragment also contains a consensus ATG sequence (M. Kozak nucleic acid Research 12:857-872 1984).

SV40 Poly-Adenylation site: The SV40-derived RNA processing elements consist of a fused fragment comprised of two noncontiguous regions of the SV40 genome. A 610 base pair region (4710 - 4100 on the SV40 map) contains an intron (66 bp) from the early region of the viral genome. This is fused to a 237 base pair fragment (2770 - 2533 on the SV40 map) containing the polyadenylation signal at position 2586. The BglII site at the 5; end was converted to an XhoI site with synthetic linkers to permit subsequent ligation. The BamHI site at the 3' end is ligated to the BamHI at the 5' end of the BPV genome to complete the circular structure. pd delta BPV/delta FVIII.ATG.c (p261-26) is 15433 base pairs in length.

## F. Expression of Factor VIII-C Analogs in Mammalian Cells

Mouse C127 cells [Lowy, D.R., et al., J. Virol., 26:72 (1978)] were maintained in Dulbecco modified Eagle medium (Meloy Laboratories, Springfield, VA ) supplemented with penicillin (10 U/ml), streptomycin (100 ug/ml), and 10% heat-inactivated fetal bovine serum (GIBCO).

DNA transformation was performed by using the calcium precipitation method (Graham and van der Eb, supra) followed by a dimethyl sulfoxide [Stowe and Wolkie, J. Gen. Virol., 33:447 (1976)] or glycerol enhancement [Frost and Williams, Virology , 91:39-50 (1978)]. Briefly, a 2X DNA-CaCl₂ solution [1 mM Tris-(mydroxymethyl) aminomethan (Tris) (pH 7.9), 0.1 mM ethylenediaminetetraacetate (EDTA), 250 mM CaCl₂, 25 ug of calf thymus DNA per ml, 2.5 ug of recombinant DNA per ml] was added to an equal volume of 2X HBS solution {1X HBS = 140 mM Nacl, 25 mM HEPES [N-2-hydroxyethylpiperazine-N'-2-ethanesulfonic acid] 0.75 mM sodium phosphate, pH 7.1), and the DNA precipitate was allowed to form at room temperature over a 45 minute period. Portions representing 1 ug of the recombinant DNA were added to cell cultures in 60-mm petri dishes containing 4 ml of fresh medium, and incubation was contained at 37°C for 4 hours. After the medium was changed, the cells were treated with either 1 ml of 25% dimethylsulfoxide in HBS for 4 minutes at room temperature or with 15% glycerol in HBS for 2 minutes at room temperature. The monolayers were washed again and refed with fresh medium.

The presence of Factor VIII-C analog cDNA in engineered cells was confirmed by Southern blot DNA analysis and the sequence verified by the dideoxy chain termination DNA sequencing technique [Sanger, et al., Proc. Natl. Acad. Sci. USA, 74:5463-5467 (1977)]. Expression of Factor VIII-C analog protein was verified by Northern blot RNA analysis and by in vitro translation of RNAs. The RNAs were either extracted from Factor VIII-C analog engineered cells or were obtained from an in vitro transcription system based on the SP6 method [Melton, et al., Nuc. Acids Res., 12:7035-7056 (1984)]. This was followed by immunoprecipitations, competitive immunoprecipitation of extracellular and intracellular proteins produced by recombinant cells, clotting assays, and biological activity assays.

## Deposit of Strains Useful in Practicing the Inventon

A deposit of biologically pure cultures of the following strains was made with the American Type Culture Collection, 12301 Parklawn Drive, Rockville, Maryland, the accession number indicated was assigned after successful viability testing, and the requisite fees were paid. Access to said culture will be available during pendency of the patent application to one determined by the above-mentioned Culture Collection Agency. Said culture will remain permanently available for a term of at least five years after the most recent request for the furnishing of a sample and in any case for a period of at least 30 years after the date of the deposit. Should the culture become nonviable or be inadventently destroyed, it will be replace with a viable culture-(s) of the same taxonomic description.

| Strain/Plasmid | ATCC Number | Submission Date |
|---|---|---|
| pd delta BPV/F8C-20 (p188-3) | 67111 | May 9, 1986 |

Submitted concurrently with filing of this application under the above conditions. pd delta BPV/delta F8C-ATG-C (p261-26).

## Claims

1. Human Factor VIII-C analog essentially free of other proteins of human origin.

2. Human Factor VIII-C analog of Claim 1 wherein the sequence of the gene encoding analogue is set forth in Figures 10-14.

3. Human Factor VIII-C analog of Claim 1 wherein the sequence of the gene encoding analogue is set forth in Figure 15-18.

4. Human factor VIII-C analog of Claim 1 wherein the sequence of the gene encoding analogue is set forth in Figures 19-22.

5. Human Factor VIII-C analog according to Claim 1 wherein the sequence of the gene encoding analogue is set forth in Figures 5-9 minus one or more exons or portions thereof.

6. A cDNA clone comprising the complete sequence for the analog of Claim 1.

7. A cDNA clone comprising the complete sequence for the analog of Claim 2, 3 or 4.

8. A cDNA clone comprising the complete sequence for the analog of Claim 5.

9. A self-replicating expression vector capable of expressing in an in vitro culture system the analog of claim 1. (p. 261-118)

10. A cell or cell-free, self-replicating system transformed with the vector of Claim 9.

11. A composition comprising a therapeutically effective amount of the analog of any of Claims 1 to 5 in a mixture with a pharmaceutically acceptable carrier.

12. The composition of Claim 11 suitable for parenteral administration.

13. A process for preparing Factor VIII-C analogs which comprises (a) preparing a replicable expression vector capable of expressing the DNA sequence encoding a Factor VIII-C analog in a suitable host cell or cell-free replicating system; (b) transforming said host system to obtain a recombinant host system; (c) maintaining said recombinant host system under conditions permitting expression of said Factor VIII-C analog encoding the DNA sequence to produce Factor VIII-C analog; and (d) recovering said Factor VIII-C analog.

14. A process according to Claim 1 wherein the DNA sequence encoding Factor VIII-C analog is as set forth in Figures 5-9 minus one or more exons or portions thereof.

15. A process according to Claim 1 or 2 wherein the DNA sequence encoding Factor VIII-C analog is as set forth in Figures 10-14, 15-18, or 19-22.

FIG.I

FIG.2

# FIG.3

ASSEMBLY OF FULL-LENGTH FVIII cDNA

**A**

740/741    1689/1690

| A1 | A2 | B | A3 | C1 | C2 | COMPLETE FVIII PROTEIN

Δ747-1560 aa

| A1 | A2 | A3 | C1 | C2 | TRUNCATED FVIII PROTEIN (ΔFVIII)

**B**

ATG

BamH1   EcoR1           SPH1    BamH1              TGA

FVIII cDNA

1870  2290              3936    4744

CAG AAT TCA    Δ229G-4737 nt    CTA TTG GAT CCT    ΔFVIII cDNA

EcoR1                                    BamH1

FIG.4

0 265 778

```
        10         20         30         40         50         60         70
ATGCAAATAG AGCTCTCCAC CTGCTTCTTT CTGTGCCTTT TGCGATTCTG CTTTAGTGCC ACCAGAAGAT

        80         90        100        110        120        130        140
ACTACCTGGG TGCAGTGGAA CTGTCATGGG ACTATATGCA AAGTGATCTC GGTGAGCTGC CTGTGGACGC

       150        160        170        180        190        200        210
AAGATTTCCT CCTAGAGTGC CAAAATCTTT TCCATTCAAC ACCTCAGTCG TGTACAAAAA GACTCTGTTT

       220        230        240        250        260        270        280
GTAGAATTCA CGGATCACCT TTTCAACATC GCTAAGCCAA GGCCACCCTG GATGGGTCTG CTAGGTCCTA

       290        300        310        320        330        340        350
CCATCCAGGC TGAGGTTTAT GATACAGTGG TCATTACACT TAAGAACATG GCTTCCCATC CTGTCAGTCT

       360        370        380        390        400        410        420
TCATGCTGTT GGTGTATCCT ACTGGAAAGC TTCTGAGGGA GCTGAATATG ATGATCAGAC CAGTCAAAGG

       430        440        450        460        470        480        490
GAGAAAGAAG ATGATAAAGT CTTCCCTGGT GGAAGCCATA CATATGTCTG GCAGGTCCTG AAAGAGAATG

       500        510        520        530        540        550        560
GTCCAATGGC CTCTGACCCA CTGTGCCTTA CCTACTCATA TCTTTCTCAT GTGGACCTGG TAAAAGACTT

       570        580        590        600        610        620        630
GAATTCAGGC CTCATTGGAG CCCTACTAGT ATGTAGAGAA GGGAGTCTGG CCAAGGAAAA GACACAGACC

       640        650        660        670        680        690        700
TTGCACAAAT TTATACTACT TTTTGCTGTA TTTGATGAAG GGAAAAGTTG GCACTCAGAA ACAAAGAACT

       710        720        730        740        750        760        770
CCTTGATGCA GGATAGGGAT GCTGCATCTG CTCGGGCCTG GCCTAAAATG CACACAGTCA ATGGTTATGT

       780        790        800        810        820        830        840
AAACAGGTCT CTGCCAGGTC TGATTGGATG CCACAGGAAA TCAGTCTATT GGCATGTGAT TGGAATGGGC

       850        860        870        880        890        900        910
ACCACTCCTG AAGTGCACTC AATATTCCTC GAAGGTCACA CATTTCTTGT GAGGAACCAT CGCCAGGCGT

       920        930        940        950        960        970        980
CCTTGGAAAT CTCGCCAATA ACTTTCCTTA CTGCTCAAAC ACTCTTGATG GACCTTGGAC AGTTTCTACT

       990       1000       1010       1020       1030       1040       1050
GTTTTGTCAT ATCTCTTCCC ACCAACATGA TGGCATGGAA GCTTATGTCA AAGTAGACAG CTGTCCAGAG

      1060       1070       1080       1090       1100       1110       1120
GAACCCCAAC TACGAATGAA AAATAATGAA GAAGCGGAAG ACTATGATGA TGATCTTACT GATTCTGAAA

      1130       1140       1150       1160       1170       1180       1190
TGGATGTGGT CAGGTTTGAT GATGACAACT CTCCTTCCTT TATCCAAATT CGCTCAGTTG CCAAGAAGCA

      1200       1210       1220       1230       1240       1250       1260
TCCTAAAACT TGGGTACATT ACATTGCTGC TGAAGAGGAG GACTGGGACT ATGCTCCCTT AGTCCTCGCC
```

# FIG.5

CCCGATGACA GAAGTTATAA AAGTCAATAT TTGAACAATG GCCCTCAGCG GATTGGTAGG AAGTACAAAA

AAGTCCGATT TATGGCATAC ACAGATGAAA CCTTTAAGAC TCGTGAAGCT ATTCAGCATG AATCAGGAAT

CTTGGGACCT TTACTTTATG GGGAAGTTGG AGACACACTG TTGATTATAT TTAAGAATCA AGCAAGCAGA

CCATATAACA TCTACCCTCA CGGAATCACT GATGTCCGTC CTTTGTATTC AAGGAGATTA CCAAAAGGTG

TAAAACATTT GAAGGATTTT CCAATTCTGC CAGGAGAAAT ATTCAAATAT AAATGGACAG TGACTGTAGA

AGATGGGCCA ACTAAATCAG ATCCTCGGTG CCTGACCCGC TATTACTCTA GTTCGTTAA TATGGAGAGA

GATCTAGCTT CAGGACTCAT TGGCCCTCTC CTCATCTGCT ACAAAGAATC TGTAGATCAA AGAGGAAACC

AGATAATGTC AGACAAGAGG AATGTCATCC TGTTTTCTGT ATTTGATGAG AACCGAAGCT GGTACCTCAC

AGAGAATATA CAACGCTTTC TCCCCAATCC AGCTGGAGTG CAGCTTGAGG ATCCAGAGTT CCAAGCCTCC

AACATCATGC ACAGCATCAA TGGCTATGTT TTTGATAGTT TGCAGTTGTC AGTTTGTTTG CATGAGGTGG

CATACTGGTA CATTCTAAGC ATTGGAGCAC AGACTGACTT CCTTTCTGTC TTCTTCTCTG GATATACCTT

CAAACACAAA ATGGTCTATG AAGACACACT CACCCTATTC CCATTCTCAG GAGAAACTGT CTTCATGTCG

ATGGAAAACC CAGGTCTATG GATTCTGGGG TGCCACAACT CAGACTTTCG GAACAGAGGC ATGACCGCCT

TACTGAAGGT TTCTAGTTGT GACAAGAACA CTGGTGATTA TTACGAGGAC AGTTATGAAG ATATTTCAGC

ATACTTGCTG AGTAAAAACA ATGCCATTGA ACCAAGAAGC TTCTCCCAGA ATTCAAGACA CCCTAGCACT

AGGCAAAAGC AATTTAATGC CACCACAATT CCAGAAAATG ACATAGAGAA GACTGACCCT TGGTTTGCAC

ACAGAACACC TATGCCTAAA ATACAAAATG TCTCCTCTAG TGATTTGTTG ATGCTCTTGC GACAGAGTCC

TACTCCACAT GGGCTATCCT TATCTGATCT CCAAGAAGCC AAATATGAGA CTTTTTCTGA TGATCCATCA

CCTGGAGCAA TAGACAGTAA TAACAGCCTG TCTGAAATGA CACACTTCAG GCCACAGCTC CATCACAGTC

GGGACATGGT ATTTACCCCT GAGTCAGGCC TCCAATTAAG ATTAAATGAG AAACTGGGGA CAACTGCAGC

AACAGAGTTG AAGAAACTTG ATTTCAAAGT TTCTAGTACA TCAAATAATC TGATTTCAAC AATTCCATCA

GACAATTTGG CAGCAGGTAC TGATAATACA AGTTCCTTAG GACCCCCAAG TATGCCAGTT CATTATGATA

## FIG.6

```
          2810       2820       2830       2840       2850       2860       2870
GTCAATTAGA TACCACTCTA TTTGGCAAAA AGTCATCTCC CCTTACTGAG TCTGGTGGAC CTCTGAGCTT

          2880       2890       2900       2910       2920       2930       2940
GAGTGAAGAA AATAATGATT CAAAGTTGTT AGAATCAGGT TTAATGAATA GCCAAGAAAG TTCATGGGGA

          2950       2960       2970       2980       2990       3000       3010
AAAAATGTAT CGTCAACAGA GAGTGGTAGG TTATTTAAAG GGAAAAGAGC TCATGGACCT GCTTTGTTGA

          3020       3030       3040       3050       3060       3070       3080
CTAAAGATAA TGCCTTATTC AAAGTTAGCA TCTCTTTGTT AAAGACAAAC AAAACTTCCA ATAATTCAGC

          3090       3100       3110       3120       3130       3140       3150
AACTAATAGA AAGACTCACA TTGATGGCCC ATCATTATTA ATTGAGAATA GTCCATCAGT CTGGCAAAAT

          3160       3170       3180       3190       3200       3210       3220
ATATTAGAAA GTGACACTGA GTTTAAAAAA GTGACACCTT TGATTCATGA CAGAATGCTT ATGGACAAAA

          3230       3240       3250       3260       3270       3280       3290
ATGCTACAGC TTTGAGGCTA AATCATATGT CAAATAAAAC TACTTCATCA AAAAACATGG AAATGGTCCA

          3300       3310       3320       3330       3340       3350       3360
ACAGAAAAAA GAGGGCCCCA TTCCACCAGA TGCACAAAAT CCAGATATGT CGTTCTTTAA GATGCTATTC

          3370       3380       3390       3400       3410       3420       3430
TTGCCAGAAT CAGCAAGGTG GATACAAAGG ACTCATGGAA AGAACTCTCT GAACTCTGGG CAAGGCCCCA

          3440       3450       3460       3470       3480       3490       3500
GTCCAAAGCA ATTAGTATCC TTAGGACCAG AAAAATCTGT GGAAGGTCAG AATTTCTTGT CTGAGAAAAA

          3510       3520       3530       3540       3550       3560       3570
CAAAGTGGTA GTAGGAAAGG GTGAATTTAC AAAGGACGTA GGACTCAAAG AGATGGTTTT TCCAAGGAGC

          3580       3590       3600       3610       3620       3630       3640
AGAAACCTAT TTCTTACTAA CTTGGATAAT TTACATGAAA ATAATACACA CAATCAAGAA AAAAAAATTC

          3650       3660       3670       3680       3690       3700       3710
AGGAAGAAAT AGAAAGAAG GAAACATTAA TCCAAGAGAA TGTAGTTTTG CCTCAGATAC ATACAGTGAC

          3720       3730       3740       3750       3760       3770       3780
TGGCACTAAG AATTTCATGA AGAACCTTTT CTTACTGAGC ACTAGGCAAA ATGTAGAAGG TTCATATGAC

          3790       3800       3810       3820       3830       3840       3850
GGGGCATATG CTCCAGTACT TCAAGATTTT AGGTCATTAA ATGATTCAAC AAATAGAACA AAGAAACACA

          3860       3870       3880       3890       3900       3910       3920
CAGCTCATTT CTCAAAAAAA GGGGAGGAAG AAAACTTGGA AGGCTTGGGA AATCAAACCA AGCAAATTGT

          3930       3940       3950       3960       3970       3980       3990
AGAGAAATAT GCATGCACCA CAAGGATATC TCCTAATACA AGCCAGCAGA ATTTTGTCAC GCAACGTAGT

          4000       4010       4020       4030       4040       4050       4060
AAGAGAGCTT TGAAACAATT CAGACTCCCA CTAGAAGAAA CAGAACTTGA AAAAAGGATA ATTGTGGATG

          4070       4080       4090       4100       4110       4120       4130
ACACCTCAAC CCAGTGGTCC AAAAACATGA AACATTTGAC CCCGAGCACC CTCACACAGA TAGACTACAA

          4140       4150       4160       4170       4180       4190       4200
TGAGAAGGAG AAAGGGGCCA TTACTCAGTC TCCCTTATCA GATTGCCTTA CGAGGAGTCA TAGCATCCCT

          4210       4220       4230       4240       4250       4260       4270
CAAGCAAATA GATCTCCATT ACCCATTGCA AAGGTATCAT CATTTCCATC TATTAGACCT ATATATCTGA

          4280       4290       4300       4310       4320       4330       4340
CCAGGGTCCT ATTCCAAGAC AACTCTTCTC ATCTTCCAGC AGCATCTTAT AGAAAGAAAG ATTCTGGGGT
```

## FIG.7

```
        4350       4360       4370       4380       4390       4400       4410
CCAAGAAAGC AGTCATTTCT TACAAGGAGC CAAAAAAAAT AACCTTTCTT TAGCCATTCT AACCTTGGAG

        4420       4430       4440       4450       4460       4470       4480
ATGACTGGTG ATCAAAGAGA GGTTGGCTCC CTGGGGACAA GTGCCACAAA TTCAGTCACA TACAAGAAAG

        4490       4500       4510       4520       4530       4540       4550
TTGAGAACAC TGTTCTCCCG AAACCAGACT TGCCCAAAAC ATCTGGCAAA GTTGAATTGC TTCCAAAAGT

        4560       4570       4580       4590       4600       4610       4620
TCACATTTAT CAGAAGGACC TATTCCCTAC GGAAACTAGC AATGGGTCTC CTGGCCATCT GGATCTCGTG

        4630       4640       4650       4660       4670       4680       4690
GAAGGGAGCC TTCTTCAGGG AACAGAGGGA GCGATTAAGT GGAATGAAGC AAACAGACCT CGAAAAGTTC

        4700       4710       4720       4730       4740       4750       4760
CCTTTCTGAG AGTAGCAACA GAAAGCTCTG CAAAGACTCC CTCCAAGCTA TTGGATCCTC TTGCTTGGGA

        4770       4780       4790       4800       4810       4820       4830
TAACCACTAT GGTACTCAGA TACCAAAAGA AGAGTGGAAA TCCCAAGAGA AGTCACCAGA AAAAACAGCT

        4840       4850       4860       4870       4880       4890       4900
TTTAAGAAAA AGGATACCAT TTTGTCCCTG AACGCTTGTG AAAGCAATCA TGCAATAGCA GCAATAAATG

        4910       4920       4930       4940       4950       4960       4970
AGGGACAAAA TAAGCCCGAA ATAGAAGTCA CCTGGGCAAA GCAAGGTAGG ACTGAAAGGC TGTGCTCTCA

        4980       4990       5000       5010       5020       5030       5040
AAACCCACCA GTCTTGAAAC GCCATCAACG GGAAATAACT CGTACTACTC TTCAGTCAGA TCAAGAGGAA

        5050       5060       5070       5080       5090       5100       5110
ATTGACTATG ATGATACCAT ATCAGTTGAA ATGAAGAAGG AAGATTTTGA CATTTATGAT GAGGATGAAA

        5120       5130       5140       5150       5160       5170       5180
ATCAGAGCCC CCGCAGCTTT CAAAAGAAAA CACGACACTA TTTTATTGCT GCAGTGGAGA GGCTCTGGGA

        5190       5200       5210       5220       5230       5240       5250
TTATGGGATG AGTAGCTCCC CACATGTTCT AAGAAACAGG GCTCAGAGTG GCAGTGTCCC TCAGTTCAAG

        5260       5270       5280       5290       5300       5310       5320
AAAGTTGTTT TCCAGGAATT TACTGATGGC TCCTTTACTC AGCCCTTATA CCGTGGAGAA CTAAATGAAC

        5330       5340       5350       5360       5370       5380       5390
ATTTGGGACT CCTGGGGCCA TATATAAGAG CAGAAGTTGA AGATAATATC ATGGTAACTT TCAGAAATCA

        5400       5410       5420       5430       5440       5450       5460
GGCCTCTCGT CCCTATTCCT TCTATTCTAG CCTTATTTCT TATGAGGAAG ATCAGAGGCA AGGAGCAGAA

        5470       5480       5490       5500       5510       5520       5530
CCTAGAAAAA ACTTTGTCAA GCCTAATGAA ACCAAAACTT ACTTTTGGAA AGTGCAACAT CATATGGCAC

        5540       5550       5560       5570       5580       5590       5600
CCACTAAAGA TGAGTTTGAC TGCAAAGCCT GGGCTTATTT CTCTGATGTT GACCTGGAAA AAGATGTGCA

        5610       5620       5630       5640       5650       5660       5670
CTCAGGCCTG ATTGGACCCC TTCTGGTCTG CCACACTAAC ACACTGAACC CTGCTCATGG GAGACAAGTG

        5680       5690       5700       5710       5720       5730       5740
ACAGTACAGG AATTTGCTCT GTTTTTCACC ATCTTTGATC AGACCAAAAG CTGGTACTTC ACTGAAAATA

        5750       5760       5770       5780       5790       5800       5810
TGGAAAGAAA CTGCAGGGCT CCCTGCAATA TCCAGATGGA AGATCCCACT TTTAAAGAGA ATTATCGCTT

        5820       5830       5840       5850       5860       5870       5880
CCATGCAATC AATGGCTACA TAATGGATAC ACTACCTGGC TTAGTAATGG CTCAGGATCA AAGGATTCGA
```

## FIG.8

```
          5890       5900       5910       5920       5930       5940       5950
TGGTATCTGC TCAGCATGGG CAGCAATGAA AACATCCATT CTATTCATTT CAGTGGACAT GTGTTCACTG

          5960       5970       5980       5990       6000       6010       6020
TACGAAAAAA AGAGGAGTAT AAAATGGCAC TGTACAATCT CTATCCAGGT GTTTTTGAGA CAGTGGAAAT

          6030       6040       6050       6060       6070       6080       6090
GTTACCATCC AAAGCTGGAA TTTGGCGGGT GGAATGCCTT ATTGGCGAGC ATCTACATGC TGGGATGAGC

          6100       6110       6120       6130       6140       6150       6160
ACACTTTTTC TGGTGTACAG CAATAAGTGT CAGACTCCCC TGGGAATGGC TTCTGGACAC ATTAGAGATT

          6170       6180       6190       6200       6210       6220       6230
TTCAGATTAC AGCTTCAGGA CAATATGGAC AGTGGGCCCC AAAGCTGGCC AGACTTCATT ATTCCGGATC

          6240       6250       6260       6270       6280       6290       6300
AATCAATGCC TGGAGCACCA AGGAGCCCTT TTCTTGGATC AAGGTGGATC TGTTGGCACC AATGATTATT

          6310       6320       6330       6340       6350       6360       6370
CACGGCATCA AGACCCAGGG TGCCCGTCAG AAGTTCTCCA GCCTCTACAT CTCTCAGTTT ATCATCATGT

          6380       6390       6400       6410       6420       6430       6440
ATAGTCTTGA TGGGAAGAAG TGGCAGACTT ATCGAGGAAA TTCCACTGGA ACCTTAATGG TCTTCTTTGG

          6450       6460       6470       6480       6490       6500       6510
CAATGTGGAT TCATCTGGGA TAAAACACAA TATTTTTAAC CCTCCAATTA TTGCTCGATA CATCCGTTTG

          6520       6530       6540       6550       6560       6570       6580
CACCCAACTC ATTATAGCAT TCGCAGCACT CTTCGCATGG AGTTGATGGG CTGTGATTTA AATAGTTGCA

          6590       6600       6610       6620       6630       6640       6650
GCATGCCATT GGGAATGGAG AGTAAAGCAA TATCAGATGC ACAGATTACT GCTTCATCCT ACTTTACCAA

          6660       6670       6680       6690       6700       6710       6720
TATGTTTGCC ACCTGGTCTC CTTCAAAAGC TCGACTTCAC CTCCAAGGGA GGAGTAATGC CTGGAGACCT

          6730       6740       6750       6760       6770       6780       6790
CAGGTGAATA ATCCAAAAGA GTGGCTGCAA GTGGACTTCC AGAAGACAAT GAAAGTCACA GGAGTAACTA

          6800       6810       6820       6830       6840       6850       6860
CTCAGGGAGT AAAATCTCTG CTTACCAGCA TGTATGTGAA GGAGTTCCTC ATCTCCAGCA GTCAAGATGG

          6870       6880       6890       6900       6910       6920       6930
CCATCAGTGG ACTCTCTTTT TTCAGAATGG CAAAGTAAAG GTTTTTCAGG GAAATCAAGA CTCCTTCACA

          6940       6950       6960       6970       6980       6990       7000
CCTGTGGTGA ACTCTCTAGA CCCACCGTTA CTGACTCGCT ACCTTCGAAT TCACCCCCAG AGTTGGGTGC

          7010       7020       7030       7040       7050       7060       7070
ACCAGATTGC CCTGAGGATG GAGGTTCTGG GCTGCGAGGC ACAGGACCTC TACTGAgggt ggccactgca

          7080       7090       7100       7110       7120       7130       7140
gcacctgcca ctgccgtcac ctctccctcc tcagctccag ggcagtgtcc ctccctggct tgccttctac

          7150       7160       7170       7180       7190       7200       7210
ctttgtgcta aatcctagca gacactgcct tgaagcctcc tgaattaact atcatcagtc ctgcatttct

          7220       7230       7240       7250       7260       7270
ttggtggggg gccaggaggg tgcatccaat ttaacttaac tcttacctat tttctgcagc tg
```

FIG.9

```
        10         20         30         40         50         60         70
atgcaaatag agctcTCCAC CTGCTTCTTT CTGTGCCTTT TGCGATTCTG CTTTAGTGCC ACCAGAAGAT

        80         90        100        110        120        130        140
ACTACCTGGG TGCAGTGGAA CTGTCATGGG ACTATATGCA AAGTGATCTC GGTGAGCTGC CTGTGGACGC

       150        160        170        180        190        200        210
AAGATTTCCT CCTAGAGTGC CAAAATCTTT TCCATTCAAC ACCTCAGTCG TGTACAAAAA GACTCTGTTT

       220        230        240        250        260        270        280
GTAGAATTCA CGGATCACCT TTTCAACATC GCTAAGCCAA GGCCACCCTG GATGGGTCTG CTAGGTCCTA

       290        300        310        320        330        340        350
CCATCCAGGC TGAGGTTTAT GATACAGTGG TCATTACACT TAAGAACATG GCTTCCCATC CTGTCAGTCT

       360        370        380        390        400        410        420
TCATGCTGTT GGTGTATCCT ACTGGAAAGC TTCTGAGGGG AGTCTGGCCA AGGAAAAGAC ACAGACCTTG

       430        440        450        460        470        480        490
CACAAATTTA TACTACTTTT TGCTGTATTT GATGAAGGGA AAAGTTGGCA CTCAGAAACA AAGAACTCCT

       500        510        520        530        540        550        560
TGATGCAGGA TAGGGATGCT GCATCTGCTC GGGCCTGGCC TAAAATGCAC ACAGTCAATG GTTATGTAAA

       570        580        590        600        610        620        630
CAGGTCTCTG CCAGGTCTGA TTGGATGCCA CAGGAAATCA GTCTATTGGC ATGTGATTGG AATGGGCACC

       640        650        660        670        680        690        700
ACTCCTGAAG TGCACTCAAT ATTCCTCGAA GGTCACACAT TTCTTGTGAG GAACCATCGC CAGGCGTCCT

       710        720        730        740        750        760        770
TGGAAATCTC GCCAATAACT TTCCTTACTG CTCAAACACT CTTGATGGAC CTTGGACAGT TTCTACTGTT

       780        790        800        810        820        830        840
TTGTCATATC TCTTCCCACC AACATGATGG CATGGAAGCT TATGTCAAAG TAGACAGCTG TCCAGAGGAA

       850        860        870        880        890        900        910
CCCCAACTAC GAATGAAAAA TAATGAAGAA GCGGAAGACT ATGATGATGA TCTTACTGAT TCTGAAATGG

       920        930        940        950        960        970        980
ATGTGGTCAG GTTTGATGAT GACAACTCTC CTTCCTTTAT CCAAATTCGC TCAGTTGCCA AGAAGCATCC

       990       1000       1010       1020       1030       1040       1050
TAAAACTTGG GTACATTACA TTGCTGCTGA AGAGGAGGAC TGGGACTATG CTCCCTTAGT CCTCGCCCCC

      1060       1070       1080       1090       1100       1110       1120
GATGACAGAA GTTATAAAAG TCAATATTTG AACAATGGCC CTCAGCGGAT TGGTAGGAAG TACAAAAAAG

      1130       1140       1150       1160       1170       1180       1190
TCCGATTTAT GGCATACACA GATGAAACCT TTAAGACTCG TGAAGCTATT CAGCATGAAT CAGGAATCTT

      1200       1210       1220       1230       1240       1250       1260
GGGACCTTTA CTTTATGGGG AAGTTGGAGA CACACTGTTG ATTATATTTA AGAATCAAGC AAGCAGACCA

      1270       1280       1290       1300       1310       1320       1330
TATAACATCT ACCCTCACGG AATCACTGAT GTCCGTCCTT TGTATTCAAG GAGATTACCA AAAGGTGTAA

      1340       1350       1360       1370       1380       1390       1400
AACATTTGAA GGATTTTCCA ATTCTGCCAG GAGAAATATT CAAATATAAA TGGACAGTGA CTGTAGAAGA

      1410       1420       1430       1440       1450       1460       1470
TGGGCCAACT AAAATCAGATC CTCGGTGCCT GACCCGCTAT TACTCTAGTT TCGTTAATAT GGAGAGAGAT

      1480       1490       1500       1510       1520       1530       1540
CTAGCTTCAG GACTCATTGG CCCTCTCCTC ATCTGCTACA AAGAATCTGT AGATCAAAGA GGAAACCAGA
```

# FIG.10

TAATGTCAGA CAAGAGGAAT GTCATCCTGT TTTCTGTATT TGATGAGAAC CGAAGCTGGT ACCTCACAGA

GAATATACAA CGCTTTCTCC CCAATCCAGC TGGAGTGCAG CTTGAGGATC CAGAGTTCCA AGCCTCCAAC

ATCATGCACA GCATCAATGG CTATGTTTTT GATAGTTTGC AGTTGTCAGT TTGTTTGCAT GAGGTGGCAT

ACTGGTACAT TCTAAGCATT GGAGCACAGA CTGACTTCCT TTCTGTCTTC TTCTCTGGAT ATACCTTCAA

ACACAAAATG GTCTATGAAG ACACACTCAC CCTATTCCCA TTCTCAGGAG AAACTGTCTT CATGTCGATG

GAAAACCCAG GTCTATGGAT TCTGGGGTGC CACAACTCAG ACTTTCGGAA CAGAGGCATG ACCGCCTTAC

TGAAGGTTTC TAGTTGTGAC AAGAACACTG GTGATTATTA CGAGGACAGT TATGAAGATA TTTCAGCATA

CTTGCTGAGT AAAAACAATG CCATTGAACC AAGAAGCTTC TCCCAGAATT CAAGACACCC TAGCACTAGG

CAAAAGCAAT TTAATGCCAC CACAATTCCA GAAAATGACA TAGAGAAGAC TGACCCTTGG TTTGCACACA

GAACACCTAT GCCTAAAATA CAAAATGTCT CCTCTAGTGA TTTGTTGATG CTCTTGCGAC AGAGTCCTAC

TCCACATGGG CTATCCTTAT CTGATCTCCA AGAAGCCAAA TATGAGACTT TTTCTGATGA TCCATCACCT

GGAGCAATAG ACAGTAATAA CAGCCTGTCT GAAATGACAC ACTTCAGGCC ACAGCTCCAT CACAGTGGGG

ACATGGTATT TACCCCTGAG TCAGGCCTCC AATTAAGATT AAATGAGAAA CTGGGGACAA CTGCAGCAAC

AGAGTTGAAG AAACTTGATT TCAAAGTTTC TAGTACATCA AATAATCTGA TTTCAACAAT TCCATCAGAC

AATTTGGCAG CAGGTACTGA TAATACAAGT TCCTTAGGAC CCCCAAGTAT GCCAGTTCAT TATGATAGTC

AATTAGATAC CACTCTATTT GGCAAAAAGT CATCTCCCCT TACTGAGTCT GGTGGACCTC TGAGCTTGAG

TGAAGAAAAT AATGATTCAA AGTTGTTAGA ATCAGGTTTA ATGAATAGCC AAGAAAGTTC ATGGGGAAAA

AATGTATCGT CAACAGAGAG TGGTAGGTTA TTTAAAGGGA AAAGAGCTCA TGGACCTGCT TTGTTGACTA

AAGATAATGC CTTATTCAAA GTTAGCATCT CTTTGTTAAA GACAAACAAA ACTTCCAATA ATTCAGCAAC

TAATAGAAAG ACTCACATTG ATGGCCCATC ATTATTAATT GAGAATAGTC CATCAGTCTG GCAAAATATA

TTAGAAAGTG ACACTGAGTT TAAAAAAGTG ACACCTTTGA TTCATGACAG AATGCTTATG GACAAAAATG

CTACAGCTTT GAGGCTAAAT CATATGTCAA ATAAAACTAC TTCATCAAAA AACATGGAAA TGGTCCAACA

# FIG.II

```
        3090      3100      3110      3120      3130      3140      3150
GAAAAAAGAG GGCCCCATTC CACCAGATGC ACAAAATCCA GATATGTCGT TCTTTAAGAT GCTATTCTTG

        3160      3170      3180      3190      3200      3210      3220
CCAGAATCAG CAAGGTGGAT ACAAAGGACT CATGGAAAGA ACTCTCTGAA CTCTGGGCAA GGCCCCAGTC

        3230      3240      3250      3260      3270      3280      3290
CAAAGCAATT AGTATCCTTA GGACCAGAAA AATCTGTGGA AGGTCAGAAT TTCTTGTCTG AGAAAAACAA

        3300      3310      3320      3330      3340      3350      3360
AGTGGTAGTA GGAAAGGGTG AATTTACAAA GGACGTAGGA CTCAAAGAGA TGGTTTTTCC AAGCAGCAGA

        3370      3380      3390      3400      3410      3420      3430
AACCTATTTC TTACTAACTT GGATAATTTA CATGAAAATA ATACACACAA TCAAGAAAAA AAAATTCAGG

        3440      3450      3460      3470      3480      3490      3500
AAGAAATAGA AAAGAAGGAA ACATTAATCC AAGAGAATGT AGTTTTGCCT CAGATACATA CAGTGACTGG

        3510      3520      3530      3540      3550      3560      3570
CACTAAGAAT TTCATGAAGA ACCTTTTCTT ACTGAGCACT AGGCAAAATG TAGAAGGTTC ATATGACGGG

        3580     ·3590      3600      3610      3620      3630      3640
GCATATGCTC CAGTACTTCA AGATTTTAGG TCATTAAATG ATTCAACAAA TAGAACAAAG AAACACACAG

        3650      3660      3670      3680      3690      3700      3710
CTCATTTCTC AAAAAAGGG GAGGAAGAAA ACTTGGAAGG CTTGGGAAAT CAAACCAAGC AAATTGTAGA

        3720      3730      3740      3750      3760      3770      3780
GAAATATGCA TGCACCACAA GGATATCTCC TAATACAAGC CAGCAGAATT TTGTCACGCA ACGTAGTAAG

        3790      3800      3810      3820      3830      3840      3850
AGAGCTTTGA AACAATTCAG ACTCCCACTA GAAGAAACAG AACTTGAAAA AAGGATAATT GTGGATGACA

        3860      3870      3880      3890      3900      3910      3920
CCTCAACCCA GTGGTCCAAA AACATGAAAC ATTTGACCCC GAGCACCCTC ACACAGATAG ACTACAATGA

        3930      3940      3950      3960      3970      3980      3990
GAAGGAGAAA GGGGCCATTA CTCAGTCTCC CTTATCAGAT TGCCTTACGA GGAGTCATAG CATCCCTCAA

        4000      4010      4020      4030      4040      4050      4060
GCAAATAGAT CTCCATTACC CATTGCAAAG GTATCATCAT TTCCATCTAT TAGACCTATA TATCTGACCA

        4070      4080      4090      4100      4110      4120      4130
GGGTCCTATT CCAAGACAAC TCTTCTCATC TTCCAGCAGC ATCTTATAGA AAGAAAGATT CTGGGGTCCA

        4140      4150      4160      4170      4180      4190      4200
AGAAAGCAGT CATTTCTTAC AAGGAGCCAA AAAAAATAAC CTTTCTTTAG CCATTCTAAC CTTGGAGATG

        4210      4220      4230      4240      4250      4260      4270
ACTGGTGATC AAAGAGAGGT TGGCTCCCTG GGGACAAGTG CCACAAATTC AGTCACATAC AAGAAAGTTG

        4280      4290      4300      4310      4320      4330      4340
AGAACACTGT TCTCCCGAAA CCAGACTTGC CCAAAACATC TGGCAAAGTT GAATTGCTTC CAAAAGTTCA

        4350      4360      4370      4380      4390      4400      4410
CATTTATCAG AAGGACCTAT TCCCTACGGA AACTAGCAAT GGGTCTCCTG GCCATCTGGA TCTCGTGGAA

        4420      4430      4440      4450      4460      4470      4480
GGGAGCCTTC TTCAGGGAAC AGAGGGAGCG ATTAAGTGGA ATGAAGCAAA CAGACCTGGA AAAGTTCCCT

        4490      4500      4510      4520      4530      4540      4550
TTCTGAGAGT AGCAACAGAA AGCTCTGCAA AGACTCCCTC CAAGCTATTG GATCCTCTTG CTTGGGATAA

        4560      4570      4580      4590      4600      4610      4620
CCACTATGGT ACTCAGATAC CAAAAGAAGA GTGGAAATCC CAAGAGAAGT CACCAGAAAA AACAGCTTTT
```

FIG.12

```
            4630       4640       4650       4660       4670       4680       4690
     AAGAAAAAGG ATACCATTTT GTCCCTGAAC GCTTGTGAAA GCAATCATGC AATAGCAGCA ATAAATGAGG

            4700       4710       4720       4730       4740       4750       4760
     GACAAAATAA GCCCGAAATA GAAGTCACCT GGGCAAAGCA AGGTAGGACT GAAAGGCTGT GCTCTCAAAA

            4770       4780       4790       4800       4810       4820       4830
     CCCACCAGTC TTGAAACGCC ATCAACGGGA AATAACTCGT ACTACTCTTC AGTCAGATCA AGAGGAAATT

            4840       4850       4860       4870       4880       4890       4900
     GACTATGATG ATACCATATC AGTTGAAATG AAGAAGGAAG ATTTTGACAT TTATGATGAG GATGAAAATC

            4910       4920       4930       4940       4950       4960       4970
     AGAGCCCCCG CAGCTTTCAA AAGAAAACAC GACACTATTT TATTGCTGCA GTGGAGAGGC TCTGGGATTA

            4980       4990       5000       5010       5020       5030       5040
     TGGGATGAGT AGCTCCCCAC ATGTTCTAAG AAACAGGGCT CAGAGTGGCA GTGTCCCTCA GTTCAAGAAA

            5050       5060       5070       5080       5090       5100       5110
     GTTGTTTTCC AGGAATTTAC TGATGGCTCC TTTACTCAGC CCTTATACCG TGGAGAACTA AATGAACATT

            5120       5130       5140       5150       5160       5170       5180
     TGGGACTCCT GGGGCCATAT ATAAGAGCAG AAGTTGAAGA TAATATCATG GTAACTTTCA GAAATCAGGC

            5190       5200       5210       5220       5230       5240       5250
     CTCTCGTCCC TATTCCTTCT ATTCTAGCCT TATTTCTTAT GAGGAAGATC AGAGGCAAGG AGCAGAACCT

            5260       5270       5280       5290       5300       5310       5320
     AGAAAAAACT TTGTCAAGCC TAATGAAACC AAAACTTACT TTTGGAAAGT GCAACATCAT ATGGCACCCA

            5330       5340       5350       5360       5370       5380       5390
     CTAAAGATGA GTTTGACTGC AAAGCCTGGG CTTATTTCTC TGATGTTGAC CTGGAAAAAG ATGTGCACTC

            5400       5410       5420       5430       5440       5450       5460
     AGGCCTGATT GGACCCCTTC TGGTCTGCCA CACTAACACA CTGAACCCTG CTCATGGGAG ACAAGTGACA

            5470       5480       5490       5500       5510       5520       5530
     GTACAGGAAT TTGCTCTGTT TTTCACCATC TTTGATGAGA CCAAAAGCTG GTACTTCACT GAAAATATGG

            5540       5550       5560       5570       5580       5590       5600
     AAAGAAACTG CAGGGCTCCC TGCAATATCC AGATGGAAGA TCCCACTTTT AAAGAGAATT ATCGCTTCCA

            5610       5620       5630       5640       5650       5660       5670
     TGCAATCAAT GGCTACATAA TGGATACACT ACCTGGCTTA GTAATGGCTC AGGATCAAAG GATTCGATGG

            5680       5690       5700       5710       5720       5730       5740
     TATCTGCTCA GCATGGGCAG CAATGAAAAC ATCCATTCTA TTCATTTCAG TGGACATGTG TTCACTGTAC

            5750       5760       5770       5780       5790       5800       5810
     GAAAAAAAGA GGAGTATAAA ATGGCACTGT ACAATCTCTA TCCAGGTGTT TTTGAGACAG TGGAAATGTT

            5820       5830       5840       5850       5860       5870       5880
     ACCATCCAAA GCTGGAATTT GGCGGGTGGA ATGCCTTATT GGCGAGCATC TACATGCTGG GATGAGCACA

            5890       5900       5910       5920       5930       5940       5950
     CTTTTTCTGG TGTACAGCAA TAAGTGTCAG ACTCCCCTGG GAATGGCTTC TGGACACATT AGAGATTTTC

            5960       5970       5980       5990       6000       6010       6020
     AGATTACAGC TTCAGGACAA TATGGACAGT GGGCCCCAAA GCTGGCCAGA CTTCATTATT CCGGATCAAT

            6030       6040       6050       6060       6070       6080       6090
     CAATGCCTGG AGCACCAAGG AGCCCTTTTC TTGGATCAAG GTGGATCTGT TGGCACCAAT GATTATTCAC

            6100       6110       6120       6130       6140       6150       6160
     GGCATCAAGA CCCAGGGTGC CCGTCAGAAG TTCTCCAGCC TCTACATCTC TCAGTTTATC ATCATGTATA
```

FIG.13

```
          10          20          30          40          50          60          70
ATGCAAATAG AGCTCTCCAC CTGCTTCTTT CTGTGCCTTT TGCGATTCTG CTTTAGTGCC ACCAGAAGAT

          80          90         100         110         120         130         140
ACTACCTGGG TGCAGTGGAA CTGTCATGGG ACTATATGCA AAGTGATCTC GGTGAGCTGC CTGTGGACGC

         150         160         170         180         190         200         210
AAGATTTCCT CCTAGAGTGC CAAAATCTTT TCCATTCAAC ACCTCAGTCG TGTACAAAAA GACTCTGTTT

         220         230         240         250         260         270         280
GTAGAATTCA CGGATCACCT TTTCAACATC GCTAAGCCAA GGCCACCCTG GATGGGTCTG CTAGGTCCTA

         290         300         310         320         330         340         350
CCATCCAGGC TGAGGTTTAT GATACAGTGG TCATTACACT TAAGAACATG GCTTCCCATC CTGTCAGTCT

         360         370         380         390         400         410         420
TCATGCTGTT GGTGTATCCT ACTGGAAAGC TTCTGAGGGA GCTGAATATG ATGATCAGAC CAGTCAAAGG

         430         440         450         460         470         480         490
GAGAAAGAAG ATGATAAAGT CTTCCCTGGT GGAAGCCATA CATATGTCTG GCAGGTCCTG AAAGAGAATG

         500         510         520         530         540         550         560
GTCCAATGGC CTCTGACCCA CTGTGCCTTA CCTACTCATA TCTTTCTCAT GTGGACCTGG TAAAAGACTT

         570         580         590         600         610         620         630
GAATTCAGGC CTCATTGGAC CCCTACTAGT ATGTAGAGAA GGGAGTCTGG CCAAGGAAAA GACACAGACC

         640         650         660         670         680         690         700
TTGCACAAAT TTATACTACT TTTTGCTGTA TTTGATGAAG GGAAAAGTTG GCACTCAGAA ACAAAGAACT

         710         720         730         740         750         760         770
CCTTGATGCA GGATAGGGAT GCTGCATCTG CTCGGGCCTG GCCTAAAATG CACACAGTCA ATGGTTATGT

         780         790         800         810         820         830         840
AAACAGGTCT CTGCCAGGTC TGATTGGATG CCACAGGAAA TCAGTCTATT GGCATGTGAT TGGAATGGGC

         850         860         870         880         890         900         910
ACCACTCCTG AAGTGCACTC AATATTCCTC GAAGGTCACA CATTTCTTGT GAGGAACCAT CGCCAGGCGT

         920         930         940         950         960         970         980
CCTTGGAAAT CTCGCCAATA ACTTTCCTTA CTGCTCAAAC ACTCTTGATG GACCTTGGAC AGTTTCTACT
```

# FIG.14

GTCTTGATGG GAAGAAGTGG CAGACTTATC GAGGAAATTC CACTGGAACC TTAATGGTCT TCTTTGGCAA

TGTGGATTCA TCTGGGATAA AACACAATAT TTTTAACCCT CCAATTATTG CTCGATACAT CCGTTTGCAC

CCAACTCATT ATAGCATTCG CAGCACTCTT CGCATGGAGT TGATGGGCTG TGATTTAAAT AGTTGCAGCA

TGCCATTGGG AATGGAGAGT AAAGCAATAT CAGATGCACA GATTACTGCT TCATCCTACT TTACCAATAT

GTTTGCCACC TGGTCTCCTT CAAAAGCTCG ACTTCACCTC CAAGGGAGGA GTAATGCCTG GAGACCTCAG

GTGAATAATC CAAAAGAGTG GCTGCAAGTG GACTTCCAGA AGACAATGAA AGTCACAGGA GTAACTACTC

AGGGAGTAAA ATCTCTGCTT ACCAGCATGT ATGTGAAGGA GTTCCTCATC TCCAGCAGTC AAGATGGCCA

TCAGTGGACT CTCTTTTTTC AGAATGGCAA AGTAAAGGTT TTTCAGGGAA ATCAAGACTC CTTCACACCT

GTGGTGAACT CTCTAGACCC ACCGTTACTG ACTCGCTACC TTCGAATTCA CCCCCAGAGT TGGGTGCACC

AGATTGCCCT GAGGATGGAG GTTCTGGGCT GCGAGGCACA GGACCTCTAC TGAgggtggc cactgcagca

cctgccactg ccgtcacctc tccctcctca gctccagggc agtgtccctc cctggcttgc cttctacctt

tgtgctaaat cctagcagac actgccttga agcctcctga attaactatc atcagtcctg catttctttg

gtgggggggcc aggagggtgc atccaattta acttaactct tacctatttt ctgcagctg

# FIG.15

```
        990       1000  ·    1010      1020       1030      1040      1050
GTTTTGTCAT ATCTCTTCCC ACCAACATGA TGGCATGGAA GCTTATGTCA AAGTAGACAG CTGTCCAGAG

       1060      1070      1080      1090      1100      1110      1120
GAACCCCAAC TACGAATGAA AAATAATGAA GAAGCGGAAG ACTATGATGA TGATCTTACT GATTCTGAAA

       1130      1140      1150      1160      1170      1180      1190
TGGATGTGGT CAGGTTTGAT GATGACAACT CTCCTTCCTT TATCCAAATT CGCTCAGTTG CCAAGAAGCA

       1200      1210      1220      1230      1240      1250      1260
TCCTAAAACT TGGGTACATT ACATTGCTGC TGAAGAGGAG GACTGGGACT ATGCTCCCTT AGTCCTCGCC

       1270      1280      1290      1300.     1310      1320      1330
CCCGATGACA GAAGTTATAA AAGTCAATAT TTGAACAATG GCCCTCAGCG GATTGGTAGG AAGTACAAAA

       1340      1350      1360      1370      1380      1390      1400
AAGTCCGATT TATGGCATAC ACAGATGAAA CCTTTAAGAC TCGTGAAGCT ATTCAGCATG AATCAGGAAT

       1410      1420      1430      1440      1450      1460      1470
CTTGGGACCT TTACTTTATG GGGAAGTTGG AGACACACTG TTGATTATAT TTAAGAATCA AGCAAGCAGA

       1480      1490      1500      1510      1520      1530      1540
CCATATAACA TCTACCCTCA CGGAATCACT GATGTCCGTC CTTTGTATTC AAGGAGATTA CCAAAAGGTG

       1550      1560      1570      1580      1590      1600      1610
TAAAACATTT GAAGGATTTT CCAATTCTGC CAGGAGAAAT ATTCAAATAT AAATGGACAG TGACTGTAGA

       1620      1630      1640      1650      1660      1670      1680
AGATGGGCCA ACTAAATCAG ATCCTCGGTG CCTGACCCGC TATTACTCTA GTTTCGTTAA TATGGAGAGA

       1690      1700      1710      1720      1730      1740      1750
GATCTAGCTT CAGGACTCAT TGGCCCTCTC CTCATCTGCT ACAAAGAATC TGTAGATCAA AGAGGAAACC

       1760      1770      1780      1790      1800      1810      1820
AGATAATGTC AGACAAGAGG AATGTCATCC TGTTTTCTGT ATTTGATGAG AACCGAAGCT GGTACCTCAC

       1830      1840      1850      1860      1870      1880      1890
AGAGAATATA CAACGCTTTC TCCCCAATCC AGCTGGAGTG CAGCTTGAGG ATCCAGAGTT CCAAGCCTCC

       1900      1910      1920      1930      1940      1950      1960
AACATCATGC ACAGCATCAA TGGCTATGTT TTTGATAGTT TGCAGTTGTC AGTTTGTTTG CATGAGGTGG

       1970      1980      1990      2000      2010      2020      2030
CATACTGGTA CATTCTAAGC ATTGGAGCAC AGACTGACTT CCTTTCTGTC TTCTTCTCTG GATATACCTT

       2040      2050      2060      2070      2080      2090      2100
CAAACACAAA ATGGTCTATG AAGACACACT CACCCTATTC CCATTCTCAG GAGAAACTGT CTTCATGTCG

       2110      2120      2130      2140      2150      2160      2170
ATGGAAAACC CAGGTCTATG GATTCTGGGG TGCCACAACT CAGACTTTCG GAACAGAGGC ATGACCGCCT

       2180      2190      2200      2210      2220      2230      2240
TACTGAAGGT TTCTAGTTGT GACAAGAACA CTGGTGATTA TTACGAGGAC AGTTATGAAG ATATTTCAGC

       2250      2260      2270      2280      2290      2300      2310
ATACTTGCTG AGTAAAAACA ATGCCATTGA ACCAAGAAGC TTCTCCCAGA ATTCACTATT GGATCCTCTT

       2320      2330      2340      2350      2360      2370      2380
GCTTGGGATA ACCACTATGG TACTCAGATA CCAAAAGAAG AGTGGAAATC CAAGAGAAG TCACCAGAAA

       2390      2400      2410      2420      2430      2440      2450
AAACAGCTTT TAAGAAAAAG GATACCATTT TGTCCCTGAA CGCTTGTGAA AGCAATCATG CAATAGCAGC

       2460      2470      2480      2490      2500      2510      2520
AATAAATGAG GGACAAAATA AGCCCGAAAT AGAAGTCACC TGGGCAAAGC AAGGTAGGAC TGAAAGGCTG
```

FIG.16

TGCTCTCAAA ACCCACCAGT CTTGAAACGC CATCAACGGG AAATAACTCG TACTACTCTT CAGTCAGATC

AAGAGGAAAT TGACTATGAT GATACCATAT CAGTTGAAAT GAAGAAGGAA GATTTTGACA TTTATGATGA

GGATGAAAAT CAGAGCCCCC GCAGCTTTCA AAAGAAAACA CGACACTATT TTATTGCTGC AGTGGAGAGG

CTCTGGGATT ATGGGATGAG TAGCTCCCCA CATGTTCTAA GAAACAGGGC TCAGAGTGGC AGTGTCCCTC

AGTTCAAGAA AGTTGTTTTC CAGGAATTTA CTGATGGCTC CTTTACTCAG CCCTTATACC GTGGAGAACT

AAATGAACAT TTGGGACTCC TGGGGCCATA TATAAGAGCA GAAGTTGAAG ATAATATCAT GGTAACTTTC

AGAAATCAGG CCTCTCGTCC CTATTCCTTC TATTCTAGCC TTATTTCTTA TGAGGAAGAT CAGAGGCAAG

GAGCAGAACC TAGAAAAAAC TTTGTCAAGC CTAATGAAAC CAAAACTTAC TTTTGGAAAG TGCAACATCA

TATGGCACCC ACTAAAGATG AGTTTGACTG CAAAGCCTGG GCTTATTTCT CTGATGTTGA CCTGGAAAAA

GATGTGCACT CAGGCCTGAT TGGACCCCTT CTGGTCTGCC ACACTAACAC ACTGAACCCT GCTCATGGGA

GACAAGTGAC AGTACAGGAA TTTGCTCTGT TTTTCACCAT CTTTGATGAG ACCAAAAGCT GGTACTTCAC

TGAAAATATG GAAAGAAACT GCAGGGCTCC CTGCAATATC CAGATGGAAG ATCCCACTTT TAAAGAGAAT

TATCGCTTCC ATGCAATCAA TGGCTACATA ATGGATACAC TACCTGGCTT AGTAATGGCT CAGGATCAAA

GGATTCGATG GTATCTGCTC AGCATGGGCA GCAATGAAAA CATCCATTCT ATTCATTTCA GTGGACATGT

GTTCACTGTA CGAAAAAAAG AGGAGTATAA AATGGCACTG TACAATCTCT ATCCAGGTGT TTTTGAGACA

GTGGAAATGT TACCATCCAA AGCTGGAATT TGGCGGGTGG AATGCCTTAT TGGCGAGCAT CTACATGCTG
                                                                        c
GGATGAGCAC ACTTTTTCTG GTGTACAGCA ATAAGTGTCA GACTCCCCTG GGAATGGCTT CTGGACACAT

TAGAGATTTT CAGATTACAG CTTCAGGACA ATATGGACAG TGGGCCCCAA AGCTGGCCAG ACTTCATTAT

TCCGGATCAA TCAATGCCTG GAGCACCAAG GAGCCCTTTT CTTGGATCAA GGTGGATCTG TTGGCACCAA

TGATTATTCA CGGCATCAAG ACCCAGGGTG CCCGTCAGAA GTTCTCCAGC CTCTACATCT CTCAGTTTAT

CATCATGTAT AGTCTTGATG GGAAGAAGTG GCAGACTTAT CGAGGAAATT CCACTGGAAC CTTAATGGTC

TTCTTTGGCA ATGTGGATTC ATCTGGGATA AAACACAATA TTTTTAACCC TCCAATTATT GCTCGATACA

# FIG.17

```
        4070       4080       4090       4100       4110       4120       4130
TCCGTTTGCA CCCAACTCAT TATAGCATTC GCAGCACTCT TCGCATGGAG TTGATGGGCT GTGATTTAAA

        4140       4150       4160       4170       4180       4190       4200
TAGTTGCAGC ATGCCATTGG GAATGGAGAG TAAAGCAATA TCAGATGCAC AGATTACTGC TTCATCCTAC

        4210       4220       4230       4240       4250       4260       4270
TTTACCAATA TGTTTGCCAC CTGGTCTCCT TCAAAAGCTC GACTTCACCT CCAAGGGAGG AGTAATGCCT

        4280       4290       4300       4310       4320       4330       4340
GGAGACCTCA GGTGAATAAT CCAAAAGAGT GGCTGCAAGT GGACTTCCAG AAGACAATGA AAGTCACAGG

        4350       4360       4370       4380       4390       4400       4410
AGTAACTACT CAGGGAGTAA AATCTCTGCT TACCAGCATG TATGTGAAGG AGTTCCTCAT CTCCAGCAGT

        4420       4430       4440       4450       4460       4470       4480
CAAGATGGCC ATCAGTGGAC TCTCTTTTTT CAGAATGGCA AAGTAAAGGT TTTTCAGGGA AATCAAGACT

        4490       4500       4510       4520       4530       4540       4550
CCTTCACACC TGTGGTGAAC TCTCTAGACC CACCGTTACT GACTCGCTAC CTTCGAATTC ACCCCCAGAG

        4560       4570       4580       4590       4600       4610       4620
TTGGGTGCAC CAGATTGCCC TGAGGATGGA GGTTCTGGGC TGCGAGGCAC AGGACCTCTA CTGAgggtgg

        4630       4640       4650       4660       4670       4680       4690
ccactgcagc acctgccact gccgtcacct ctccctcctc agctccaggg cagtgtccct ccctggcttg

        4700      .4710       4720       4730       4740       4750       4760
ccttctacct ttgtgctaaa tcctagcaga cactgccttg aagcctcctg aattaactat catcagtcct

        4770       4780       4790       4800       4810       4820       4830
gcatttcttt ggtgggggggc caggagggtg catccaattt aacttaactc ttacctattt tctgcagctg
```

# FIG.18

```
         10          20          30          40          50          60          70
atgcaaatag agctcTCCAC CTGCTTCTTT CTGTGCCTTT TGCGATTCTG CTTTAGTGCC ACCAGAAGAT

         80          90         100         110         120         130         140
ACTACCTGGG TGCAGTGGAA CTGTCATGGG ACTATATGCA AAGTGATCTC GGTGAGCTGC CTGTGGACGC

        150         160         170         180         190         200         210
AAGATTTCCT CCTAGAGTGC CAAAATCTTT TCCATTCAAC ACCTCAGTCG TGTACAAAAA GACTCTGTTT

        220         230         240         250         260         270         280
GTAGAATTCA CGGATCACCT TTTCAACATC GCTAAGCCAA GGCCACCCTG GATGGGTCTG CTAGGTCCTA

        290         300         310         320         330         340         350
CCATCCAGGC TGAGGTTTAT GATACAGTGG TCATTACACT TAAGAACATG GCTTCCCATC CTGTCAGTCT

        360         370         380         390         400         410         420
TCATGCTGTT GGTGTATCCT ACTGGAAAGC TTCTGAGGGG AGTCTGGCCA AGGAAAAGAC ACAGACCTTG

        430         440         450         460         470         480         490
CACAAATTTA TACTACTTTT TGCTGTATTT GATGAAGGGA AAAGTTGGCA CTCAGAAACA AAGAACTCCT

        500         510         520         530         540         550         560
TGATGCAGGA TAGGGATGCT GCATCTGCTC GGGCCTGGCC TAAAATGCAC ACAGTCAATG GTTATGTAAA

        570         580         590         600         610         620         630
CAGGTCTCTG CCAGGTCTGA TTGGATGCCA CAGGAAATCA GTCTATTGGC ATGTGATTGG AATGGGCACC

        640         650         660         670         680         690         700
ACTCCTGAAG TGCACTCAAT ATTCCTCGAA GGTCACACAT TTCTTGTGAG GAACCATCGC CAGGCGTCCT

        710         720         730         740         750         760         770
TGGAAATCTC GCCAATAACT TTCCTTACTG CTCAAACACT CTTGATGGAC CTTGGACAGT TTCTACTGTT

        780         790         800         810         820         830         840
TTGTCATATC TCTTCCCACC AACATGATGG CATGGAAGCT TATGTCAAAG TAGACAGCTG TCCAGAGGAA

        850         860         870         880         890         900         910
CCCCAACTAC GAATGAAAAA TAATGAAGAA GCGGAAGACT ATGATGATGA TCTTACTGAT TCTGAAATGG

        920         930         940         950         960         970         980
ATGTGGTCAG GTTTGATGAT GACAACTCTC CTTCCTTTAT CCAAATTCGC TCAGTTGCCA AGAAGCATCC

        990        1000        1010        1020        1030        1040        1050
TAAAACTTGG GTACATTACA TTGCTGCTGA AGAGGAGGAC TGGGACTATG CTCCCTTAGT CCTCGCCCCC

       1060        1070        1080        1090        1100        1110        1120
GATGACAGAA GTTATAAAAG TCAATATTTG AACAATGGCC CTCAGCGGAT TGGTAGGAAG TACAAAAAAG
```

# FIG.19

```
       1130       1140       1150       1160       1170       1180       1190
TCCGATTTAT GGCATACACA GATGAAACCT TTAAGACTCG TGAAGCTATT CAGCATGAAT CAGGAATCTT

       1200       1210       1220       1230       1240       1250       1260
GGGACCTTTA CTTTATGGGG AAGTTGGAGA CACACTGTTG ATTATATTTA AGAATCAAGC AAGCAGACCA

       1270       1280       1290       1300       1310       1320       1330
TATAACATCT ACCCTCACGG AATCACTGAT GTCCGTCCTT TGTATTCAAG GAGATTACCA AAAGGTGTAA

       1340       1350       1360       1370       1380       1390       1400
AACATTTGAA GGATTTTCCA ATTCTGCCAG GAGAAATATT CAAATATAAA TGGACAGTGA CTGTAGAAGA

       1410       1420       1430       1440       1450       1460       1470
TGGGCCAACT AAATCAGATC CTCGGTGCCT GACCCGCTAT TACTCTAGTT TCGTTAATAT GGAGAGAGAT

       1480       1490       1500       1510       1520       1530       1540
CTAGCTTCAG GACTCATTGG CCCTCTCCTC ATCTGCTACA AAGAATCTGT AGATCAAAGA GGAAACCAGA

       1550       1560       1570       1580       1590       1600       1610
TAATGTCAGA CAAGAGGAAT GTCATCCTGT TTTCTGTATT TGATGAGAAC CGAAGCTGGT ACCTCACAGA

       1620       1630       1640       1650       1660       1670       1680
GAATATACAA CGCTTTCTCC CCAATCCAGC TGGAGTGCAG CTTGAGGATC CAGAGTTCCA AGCCTCCAAC

       1690       1700       1710       1720       1730       1740       1750
ATCATGCACA GCATCAATGG CTATGTTTTT GATAGTTTGC AGTTGTCAGT TTGTTTGCAT GAGGTGGCAT

       1760       1770       1780       1790       1800       1810       1820
ACTGGTACAT TCTAAGCATT GGAGCACAGA CTGACTTCCT TTCTGTCTTC TTCTCTGGAT ATACCTTCAA

       1830       1840       1850       1860       1870       1880       1890
ACACAAAATG GTCTATGAAG ACACACTCAC CCTATTCCCA TTCTCAGGAG AAACTGTCTT CATGTCGATG

       1900       1910       1920       1930       1940       1950       1960
GAAAACCCAG GTCTATGGAT TCTGGGGTGC CACAACTCAG ACTTTCGGAA CAGAGGCATG ACCGCCTTAC

       1970       1980       1990       2000       2010       2020       2030
TGAAGGTTTC TAGTTGTGAC AAGAACACTG GTGATTATTA CGAGGACAGT TATGAAGATA TTTCAGCATA

       2040       2050       2060       2070       2080       2090       2100
CTTGCTGAGT AAAAACAATG CCATTGAACC AAGAAGCTTC TCCCAGaatt cactattgga TCCTCTTGCT

       2110       2120       2130       2140       2150       2160       2170
TGGGATAACC ACTATGGTAC TCAGATACCA AAAGAAGAGT GGAAATCCCA AGAGAAGTCA CCAGAAAAAA

       2180       2190       2200       2210       2220       2230       2240
CAGCTTTTAA GAAAAAGGAT ACCATTTTGT CCCTGAACGC TTGTGAAAGC AATCATGCAA TAGCAGCAAT

       2250       2260       2270       2280       2290       2300       2310
AAATGAGGGA CAAAATAAGC CCGAAATAGA AGTCACCTGG GCAAAGCAAG GTAGGACTGA AAGGCTGTGC

       2320       2330       2340       2350       2360       2370       2380
TCTCAAAACC CACCAGTCTT GAAACGCCAT CAACGGGAAA TAACTCGTAC TACTCTTCAG TCAGATCAAG

       2390       2400       2410       2420       2430       2440       2450
AGGAAATTGA CTATGATGAT ACCATATCAG TTGAAATGAA GAAGGAAGAT TTTGACATTT ATGATGAGGA

       2460       2470       2480       2490       2500       2510       2520
TGAAAATCAG AGCCCCCGCA GCTTTCAAAA GAAAACACGA CACTATTTTA TTGCTGCAGT GGAGAGGCTC

       2530       2540       2550       2560       2570       2580       2590
TGGGATTATG GGATGAGTAG CTCCCCACAT GTTCTAAGAA ACAGGGCTCA GAGTGGCAGT GTCCCTCAGT

       2600       2610       2620       2630       2640       2650       2660
TCAAGAAAGT TGTTTTCCAG GAATTTACTG ATGGCTCCTT TACTCAGCCC TTATACCGTG GAGAACTAAA
```

# FIG.20

```
        2670       2680       2690       2700       2710       2720       2730
TGAACATTTG GGACTCCTGG GGCCATATAT AAGAGCAGAA GTTGAAGATA ATATCATGGT AACTTTCAGA

        2740       2750       2760       2770       2780       2790       2800
AATCAGGCCT CTCGTCCCTA TTCCTTCTAT TCTAGCCTTA TTTCTTATGA GGAAGATCAG AGGCAAGGAG

        2810       2820       2830       2840       2850       2860       2870
CAGAACCTAG AAAAAACTTT GTCAAGCCTA ATGAAACCAA AACTTACTTT TGGAAAGTGC AACATCATAT

        2880       2890       2900       2910       2920       2930       2940
GGCACCCACT AAAGATGAGT TTGACTGCAA AGCCTGGGCT TATTTCTCTG ATGTTGACCT GGAAAAAGAT

        2950       2960       2970       2980       2990       3000       3010
GTGCACTCAG GCCTGATTGG ACCCCTTCTG TCTGCCACA CTAACACACT GAACCCTGCT CATGGGAGAC

        3020       3030       3040       3050       3060       3070       3080
AAGTGACAGT ACAGGAATTT GCTCTGTTTT TCACCATCTT TGATGAGACC AAAAGCTGGT ACTTCACTGA

        3090       3100       3110       3120       3130       3140       3150
AAATATGGAA AGAAACTGCA GGGCTCCCTG CAATATCCAG ATGGAAGATC CCACTTTTAA AGAGAATTAT

        3160       3170       3180       3190       3200       3210       3220
CGCTTCCATG CAATCAATGG CTACATAATG GATACACTAC CTGGCTTAGT AATGGCTCAG GATCAAAGGA

        3230       3240       3250       3260       3270       3280       3290
TTCGATGGTA TCTGCTCAGC ATGGGCAGCA ATGAAAACAT CCATTCTATT CATTTCAGTG GACATGTGTT

        3300       3310       3320       3330       3340       3350       3360
CACTGTACGA AAAAAGAGG AGTATAAAAT GGCACTGTAC AATCTCTATC CAGGTGTTTT TGAGACAGTG

        3370       3380       3390       3400       3410       3420       3430
GAAATGTTAC CATCCAAAGC TGGAATTTGG CCGGTGGAAT GCCTTATTGG CGAGCATCTA CATGCTGGGA

        3440       3450       3460       3470       3480       3490       3500
TGAGCACACT TTTTCTGGTG TACAGCAATA AGTGTCAGAC TCCCCTGGGA ATGGCTTCTG GACACATTAG

        3510       3520       3530       3540       3550       3560       3570
AGATTTTCAG ATTACAGCTT CAGGACAATA TGGACAGTGG GCCCCAAAGC TGGCCAGACT TCATTATTCC

        3580       3590       3600       3610       3620       3630       3640
GGATCAATCA ATGCCTGGAG CACCAAGGAG CCCTTTTCTT GGATCAAGGT GGATCTGTTG GCACCAATGA

        3650       3660       3670       3680       3690       3700       3710
TTATTCACGG CATCAAGACC CAGGGTGCCC GTCAGAAGTT CTCCAGCCTC TACATCTCTC AGTTTATCAT

        3720       3730       3740       3750       3760       3770       3780
CATGTATAGT CTTGATGGGA AGAAGTGGCA GACTTATCGA GGAAATTCCA CTGGAACCTT AATGGTCTTC

        3790       3800       3810       3820       3830       3840       3850
TTTGGCAATG TGGATTCATC TGGGATAAAA CACAATATTT TTAACCCTCC AATTATTGCT CGATACATCC

        3860       3870       3880       3890       3900       3910       3920
GTTTGCACCC AACTCATTAT AGCATTCGCA GCACTCTTCG CATGGAGTTG ATGGGCTGTG ATTTAAATAG

        3930       3940       3950       3960       3970       3980       3990
TTGCAGCATG CCATTGGGAA TGGAGAGTAA AGCAATATCA GATGCACAGA TTACTGCTTC ATCCTACTTT

        4000       4010       4020       4030       4040       4050       4060
ACCAATATGT TTGCCACCTG GTCTCCTTCA AAAGCTCGAC TTCACCTCCA AGGGAGGAGT AATGCCTGGA

        4070       4080       4090       4100       4110       4120       4130
GACCTCAGGT GAATAATCCA AAAGAGTGGC TGCAAGTGGA CTTCCAGAAG ACAATGAAAG TCACAGGAGT

        4140       4150       4160       4170       4180       4190       4200
AACTACTCAG GGAGTAAAAT CTCTGCTTAC CAGCATGTAT GTGAAGGAGT TCCTCATCTC CAGCAGTCAA
```

FIG.21

```
        4210        4220        4230        4240        4250        4260        4270
GATGGCCATC AGTGGACTCT CTTTTTTCAG AATGGCAAAG TAAAGGTTTT TCAGGGAAAT CAAGACTCCT

        4280        4290        4300        4310        4320        4330        4340
TCACACCTGT GGTGAACTCT CTAGACCCAC CGTTACTGAC TCGCTACCTT CGAATTCACC CCCAGAGTTG

        4350        4360        4370        4380        4390        4400        4410
GGTGCACCAG ATTGCCCTGA GGATGGAGGT TCTGGGCTGC GAGGCACAGG ACCTCTACTG Agggtggcca

        4420        4430        4440        4450        4460        4470        4480
ctgcagcaaa tgccactgcc gtcacctctc cctcctcagc tccagggcag tgtccctccc tggcttgcct

        4490        4500        4510        4520        4530        4540        4550
tctacctttg tgctaaatcc tagcagacac tgccttgaag cctcctgaat taactatcat cagtcctgca

        4560        4570        4580        4590        4600        4610
tttctttggt gggggggccag gagggtgcat ccaatttaac ttaactctta cctattttct gcagctg
```

# FIG.22

FIG.23

Aat II   Xho I             SAC I

```
CCAAGCTTCTCGAGAACATTTGTAGCAAT CCACCATG GGAATAGAGCT
TGCAGGTTCGAAGAGCTCTTGTAAACATCGTTA GGTGGTAC CCTTATC
```

ATC.G

FIG. 24

0 265 778

FIG. 25

0 265 778

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | PROCEEDINGS OF THE NATIONAL ACADEMY OF SCIENCE OF THE USA, vol. 83, August 1986, pages 5939-5942; J.J. TOOLE et al.: "A large region ($\approx$95kDa) of human factor VIII is dispensable for in vitro procoagulant activity" * Whole document * | 1,5,6,8 -14 | C 12 N 15/00 C 12 N 1/00 C 12 P 21/02 A 61 K 37/02 C 07 K 13/00 |
| Y | --- | 3,7,15 | |
| P,X | WO-A-8 606 101 (GENETICS INSTITUTE) * Whole document * | 1,5,6,8 -14 | |
| P,Y | --- | 3,7,15 | |
| P,X | BIOCHEMISTRY, vol. 25, no. 26, 30th December 1986, pages 8343-8347, American Chemical Society, Washington, DC, US; D.L. EATON et al.: "Construction and characterization of an active factor VIII variant lacking the central one-third of the molecule" * Whole document * | 1,5,6,8 -14 | |
| P,Y | --- | 3,7,15 | **TECHNICAL FIELDS SEARCHED (Int. Cl.4)** C 12 N C 12 P |
| E | EP-A-0 253 455 (GIST BROCADES) * Examples 11,14; figures 11,17 * | 1,5,6,8 -14 | A 61 K C 07 K |
| A | --- THE JOURNAL OF BIOLOGICAL CHEMISTRY, vol. 261, no. 27, 25th September 1986, pages 12574-12578, American Society of Biological Chemists, Inc., US; R.L. BURKE et al.: "The functional domains of coagulation factor VIII:C" * Whole document * --- | 1,3,5- 15 | |

-/-

The present search report has been drawn up for all claims

| Place of search | Date of completion of the search | Examiner |
|---|---|---|
| THE HAGUE | 28-01-1988 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

**EUROPEAN SEARCH REPORT**

Application Number

EP 87 11 5043

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 150 735 (CHIRON CORP.)<br>* Whole document * <br>----- | 1,3,5-15 | |

TECHNICAL FIELDS SEARCHED (Int. Cl.4)

The present search report has been drawn up for all claims

| Place of search | Date of completion of the search | Examiner |
|---|---|---|
| THE HAGUE | 28-01-1988 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)